# EUROPEAN PATENT APPLICATION

(11) **EP 1 953 170 A1**
(43) Date of publication of application: **06.08.2008**
(21) Application number: 07117842.0
(22) Date of filing: 03.10.2007
(51) Int. Cl.: C07H 15/00, C07H 9/04, C07H 3/02, A61K 31/70, A61K 31/7004, A61K 31/7028, A61K 31/7042, A61P 35/00, A61P 37/00, A61P 17/00, A61P 11/06

(54) **Monosaccharide derivatives as anti-inflammatory agents**

(30) Priority: 03.10.2006 IN DE21772006; 31.10.2006 IN DE23602006
(71) Applicant: Ranbaxy Laboratories Limited, Haryana 122001, Delhi (IN)
(72) Inventor: Verma, Ashwani Kumar, 110018, Dehli (IN); Malhotra, Sanjay, 110075, New Dehli (IN); Dharmarajan, Sankaranarayanan, 122001, Haryana (IN); Shirumalla, Rajkumar, 110018, New Dehli (IN); Ray, Abhijit, 110070, New Dehli (IN)
(74) Representative: Cronin, Brian Harold John

(57) **Abstract**

The present invention relates to monosaccharide derivatives as anti-inflammatory agents. The compounds disclosed herein can be useful for inhibition and prevention of inflammation and associated pathologies including inflammatory, cancer, cardiovascular and autoimmune diseases such as bronchial asthma, rheumatoid arthritis, type-I diabetes, multiple sclerosis, allograft rejection, psoriasis, inflammatory bowel disease, ulcerative colitis, acne, atherosclerosis, pruritis or allergic rhinitis. Pharmacological compositions containing compounds disclosed herein and the methods of treating diseases such as bronchial asthma, rheumatoid arthritis, type-I diabetes, multiple sclerosis, cancer, cardiovascular diseases, allograft rejection, psoriasis, inflammatory bowel disease, ulcerative colitis, acne, atherosclerosis, pruritis or allergic rhinitis and other inflammatory and/or autoimmune disorders, using the compounds are also provided.

## Description

### Field of the Invention

The present invention relates to monosaccharide derivatives as anti-inflammatory agents. The compounds disclosed herein can be useful for inhibition and prevention of inflammation and associated pathologies including inflammatory, cancer, cardiovascular and autoimmune diseases such as bronchial asthma, rheumatoid arthritis, type-I diabetes, multiple sclerosis, allograft rejection, psoriasis, inflammatory bowel disease, ulcerative colitis, acne, atherosclerosis, pruritis or allergic rhinitis. Pharmacological compositions containing compounds disclosed herein and the methods of treating diseases such as bronchial asthma, rheumatoid arthritis, type-I diabetes, multiple sclerosis, cancer, cardiovascular diseases, allograft rejection, psoriasis, inflammatory bowel disease, ulcerative colitis, acne, atherosclerosis, pruritis or allergic rhinitis and other inflammatory and/or autoimmune disorders, using the compounds are also provided.

### Background of the Invention

Inflammation is a key defence mechanism of the body that is activated as a result of tissue injury. The inflammatory process is self-containing, however, under certain pathophysiological conditions the inflammatory process tends to perpetuate itself, giving rise to chronic inflammatory diseases like bronchial asthma, rheumatoid arthritis etc.

Although the exact cellular and molecular basis of most chronic inflammatory disease remain unclear, it has become apparent that several inflammatory cells act in concert towards initiation and perpetuation of an inflammatory response by releasing a wide range of chemokine, cytokine, proteolytic enzymes and other bioactive molecules. Mast cells primed by lymphocytes interact with environmental allergens and release mediators like histamine, prostaglandin, leukotriene etc *(*Clin. Exp. Allergy 32, 1682, 2002) to initiate an early inflammatory response. This is followed by a delayed inflammatory response due to release of cytokines (IL-4, IL-5, IL-6, IL-8, IL-13, GM-CSF and TNF-alpha), chemokines and proteolytic enzymes (chymase, tryptase) (Chest 112, 523, 1997; Lancet 350, 59, 1997) that not only bring about tissue damage, but attract other inflammatory cells and initiate tissue fibrosis, and the cycle continues. Eosinophils infiltrate inflamed tissue following allergen-mast cell interaction in bronchial asthma and allergic rhinitis. Evidence is emerging that mast cells also interact with bacterial endotoxins leading to generation of cytokines like TNF-alpha, that encourage neutrophil influx into the site of inflammation (Br. J. Pharmacol. 123, 31, 1998; Br. J. Pharmacol. 128, 700, 1999; Br. J. Pharmacol. 136, 111, 2002; J. Clin. Invest. 109, 1351, 2002). Involvement of mast cells in the inflammatory response of chronic obstructive pulmonary disease.
(New Eng. J. Med. 347, 1040, 2002; Thorax 57, 649, 2002), inflammatory bowel disease (Gut. 45 Suppl. II6, 1999) as well as in rheumatoid arthritis *(*Science 297, 1626, 2002), pathologies with prominent neutrophilic inflammation, has been proposed.

U.S. Patent 6,329,344 discloses several monosaccharide derivatives said to be useful as cell adhesion inhibitors, generally related to substituted pentose and hexose monosaccharide derivatives, which are said to exhibit cell adhesion inhibitory and anti-inflammatory activities. U.S. Patent 6,590,085 discloses several monosaccharide derivatives described as inhibitors of cell adhesion and cell adhesion-mediated pathologies, including inflammatory and autoimmune diseases. U.S. Patent Application US 2002/0173632 discloses furanose and amino furanose compounds reportedly useful for treatment of rheumatoid arthritis, immunomodulatory diseases, inflammatory and proliferative diseases. U.S. Patent 5,298,494 discloses derivatives of monosaccharides, which are said to exhibit anti-proliferative and/or anti-inflammatory activity and are believed useful for treating mammals having inflammatory disorders and/or autoimmune disorders. U.S Patent 4,996,195 discloses derivatives of α-D-glucofuranose and α-D-allofuranose described as useful for treating animals and mammals with inflammatory and/or autoimmune disorders.

WO 93/13117 and U.S. Patent 5,360,792 disclose 5- or 6-deoxy hexose monosaccharides having a saturated nitrogen-containing heterocycle described as anti-proliferative and anti-inflammatory compounds. WO 94/28910 discloses 5, 6-dideoxy-5-amino derivatives of iodose and 6-deoxy-6-amino derivatives of glucose, which reportedly exhibit immunomodulatory, anti-inflammatory and anti-proliferative activity. WO 94/11381 discloses derivatives of pentose monosaccharides described as anti-proliferative and anti-inflammatory compounds. U.S. Patent 5,010,058 discloses 1, 2-O-isopropylidene-α-O-glucofuranoside derivatives useful for treating inflammatory and autoimmune disorders. U.S. Patent 4,849,512 discloses 3-acylamino-3-deoxyallose derivatives. U.S. Patent 5,367,062 discloses disubstituted and deoxy disubstituted derivatives of α-D-lyxofuranosides reportedly having anti-inflammatory and anti-proliferative activity. U.S. Patent 5,360,794 discloses disubstituted derivatives of α-D-mannofuranoside reportedly having anti-inflammatory and anti proliferative activity. WO 03/029263 discloses 3-deoxy-3-amide derivatives of carbohydrates described as useful as inducers of erythroid cell differentiation. FR 2735130 discloses regiospecific synthesis of new carbamic polyesters.

### Summary of the Invention

Monosaccharide derivatives which can be used for the inhibition and prevention of inflammation and associated pathologies, including inflammatory, cancer, cardiovascular and autoimmune diseases such as bronchial asthma, rheumatoid arthritis, type-I diabetes, multiple sclerosis, allograft rejection, psoriasis, inflammatory bowel disease, ulcerative colitis, acne, atherosclerosis, pruritis or allergic rhinitis are described herein. Pharmaceutically acceptable salts, pharmaceutically acceptable solvates, enantiomers, diastereomers or *N*-oxides of these compounds having the same type of activity are also provided. Pharmaceutical compositions containing the compounds, and which may also contain pharmaceutically acceptable carriers or diluents, which may be used for the treatment of inflammatory, cancer, cardiovascular and autoimmune diseases such as bronchial asthma, rheumatoid arthritis, type-I diabetes, multiple sclerosis, allograft rejection, psoriasis, inflammatory bowel disease, ulcerative colitis, acne, atherosclerosis, pruritis or allergic rhinitis are provided herein.

Other aspects will be set forth in accompanying description which follows and in part will be apparent from the description or may be learnt by the practice of the invention.

In accordance with one aspect, there are provided compounds having the structure of Formula I. 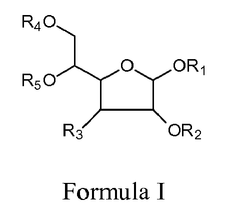 and their pharmaceutically acceptable salts, pharmaceutically acceptable solvates, esters, enantiomers, diastereomers, *N*-oxides, polymorphs, metabolites, wherein
R₁ and R₂ can together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F, Cl, Br, I), aryl, aralkyl, heteroaryl, heterocyclyl, heteroarylalkyl, or heterocyclylalkyl; or R_{L} and Rₘ can together join to form an oxo group].
R₃ can be
A) -(CH₂)ₙG wherein n is an integer from 0-5 and G is selected from
   1) NRⱼYRᵤ (wherein Rⱼ is selected from hydrogen, lower (C₁-C₆) alkyl, lower (C₂-C₆) alkenyl, lower (C₂-C₆) alkynyl, lower (C₃-C₆) cycloalkyl, aryl, heteroaryl (with the proviso that the heteroaryl ring is not linked through a heteroatom), aralkyl (C₁-C₄), heteroarylalkyl (C₁-C₄), and heterocyclylalkyl (C₁-C₄), and Y is -C(=O), -C(=S) or SO₂ and Rᵤ is selected from alkyl, alkenyl, alkynyl, cycloalkyl, aryl, aralkyl, heteroaryl, heterocyclyl, heteroarylalkyl, and heterocyclylalkyl; and when n is 0 then Y cannot be -C(=O));
   2) -NRⱼC(=T)NRₜRₓ (wherein Rₜ is OH or Rₓ and T is O, S, -N(CN), -N(NO₂), or -CH(NO₂), Rⱼ is the same as defined above and Rₓ is selected from hydrogen, alkyl, alkenyl, alkynyl, cycloalkyl, aryl, aralkyl, heteroaryl, heterocyclyl, heteroarylalkyl, heterocyclylalkyl, and -S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino);
R₄ and R₅ can independently be selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier) with the proviso that when R₃ is ORₑ then the acetal must be isopropylidene acetal.
The following definitions apply to terms as used herein.

The term "alkyl," unless otherwise specified, refers to a monoradical branched or unbranched saturated hydrocarbon chain having from 1 to 20 carbon atoms. Alkyl groups can be optionally interrupted by atom(s) or group(s) independently selected from oxygen, sulfur, a phenylene, sulphinyl, sulphonyl group or -NR_{α}-, wherein R_{α} can be hydrogen, alkyl, cycloalkyl, alkenyl, cycloalkenyl, alkynyl, aryl, acyl, aralkyl, -C(=O)OR_{λ}, SOₘR_{ψ} or -C(=O)NR_{λ}R_{π}. This term can be exemplified by groups such as methyl, ethyl, n-propyl, iso-propyl, n-butyl, iso-butyl, sec-butyl, t-butyl, n-pentyl, isopentyl, neopentyl, n-hexyl, n-decyl, tetradecyl, and the like. Alkyl groups may be substituted further with one or more substituents selected from alkenyl, alkynyl, alkoxy, cycloalkyl, cycloalkenyl, acyl, acylamino, acyloxy, alkoxycarbonylamino, azido, cyano, halogen, hydroxy, keto, oxo, thiocarbonyl, carboxy, carboxyalkyl, aryl, heterocyclyl, heteroaryl, (heterocyclyl)alkyl, cycloalkoxy, -CH=N-O(C₁₋₆alkyl), -CH=N-NH(C₁₋₆alkyl), -CH=N-NH(C₁₋₆alkyl)-C₁₋₆alkyl, arylthio, thiol, alkylthio, aryloxy, nitro, aminosulfonyl, aminocarbonylamino, -NHC(=O)R_{λ}, -NR_{λ}R_{π}, -C(=O)NR_{λ}R_{π}, -NHC(=O)NR_{λ}R_{π}, -C(=O)heteroaryl, C(=O)heterocyclyl, -O-C(=O)NR_{λ}R_{π} {wherein R_{λ} and R_{π} are independently selected from hydrogen, halogen, hydroxy, alkyl, alkenyl, alkynyl, alkenyl, alkoxy, cycloalkyl, cycloalkenyl, aryl, aralkyl, heterocyclyl, heteroaryl, heterocyclylalkyl, heteroarylalkyl or carboxy}, nitro or -SOₘR_{ψ} (wherein m is an integer from 0-2 and R_{ψ} is hydrogen, alkyl, alkenyl, alkynyl, cycloalkyl, aralkyl, aryl, heterocyclyl, heteroaryl, heteroarylalkyl or heterocyclylalkyl). Unless otherwise constrained by the definition, alkyl substituents may be further substituted by 1-3 substituents selected from alkyl, alkenyl, alkynyl, carboxy, -NR_{λ},R_{π}, -C(=O)NR_{λ}R_{π},-OC(=O)NR_{λ}R_{π},-NHC(=O)NR_{λ}R_{π}, hydroxy, alkoxy, halogen, CF₃, cyano, and -SOₘR_{ψ}; or an alkyl group also may be interrupted by 1-5 atoms of groups independently selected from oxygen, sulfur or -NR_{α}- (wherein R_{α}, R_{λ}, R_{π}, m and R_{ψ}, are the same as defined earlier). Unless otherwise constrained by the definition, all substituents may be substituted further by 1-3 substituents selected from alkyl, alkenyl, alkynyl, carboxy, carboxyalkyl, -NR_{λ}R_{π}, -C(=O)NR_{λ}R_{π}, -O-C(=O)NR_{λ}R_{π}, hydroxy, alkoxy, halogen, CF₃, cyano, and -SOₘR_{ψ} (wherein R_{λ}, R_{π}, m and R_{ψ}, are the same as defined earlier); or an alkyl group as defined above that has both substituents as defined above and is also interrupted by 1-5 atoms or groups as defined above.

The term "alkylene," as used herein, refers to a diradical branched or unbranched saturated hydrocarbon chain having from 1 to 6 carbon atoms and one or more hydrogen can optionally be substituted with alkyl, hydroxy, halogen or oximes. This term can be exemplified by groups such as methylene, ethylene, propylene isomers (*e.g*., -CH₂CH₂CH₂ and -CH(CH₃)CH₂) and the like. Alkylene may further be substituted with one or more substituents such as alkyl, alkenyl, alkynyl, alkoxy, cycloalkyl, acyl, acylamino, acyloxy, alkoxycarbonylamino, azido, cyano, halogen, hydroxy, oxo, thiocarbonyl, carboxy, arylthio, thiol, alkylthio, aryloxy, heteroaryloxy, aminosulfonyl, -COOR_{ψ}, -NHC(=O)R_{λ}, -NR_{λ}R_{π}, -C(=O)NR_{λ}R_{π}, -NHC(=O)NR_{λ}R_{π}, -C(=O)heteroaryl, C(=O)heterocyclyl, -O-C(=O)NR_{λ}R_{π}, nitro, -S(O)ₘR_{λ} (wherein R_{λ}, R_{π}, m and R_{ψ} are the same as defined earlier). Unless otherwise constrained by the definition, all substituents may be further substituted by 1-3 substituents chosen from alkyl, alkenyl, alkynyl, carboxy, -COOR_{ψ}, -NR_{λ}R_{π}, -C(=O)NR_{λ}R_{π}, -OC(=O)NR_{λ}R_{π}, -NHC(=O)NR_{λ}R_{π}, hydroxy, alkoxy, halogen, CF₃, cyano, and -S(O)ₘR_{ψ} (wherein R_{λ}, R_{π}, m and R_{ψ} are the same as defined earlier). Alkylene can also be optionally interrupted by 1-5 atoms of groups independently chosen from oxygen, sulfur and -NR_{α} (wherein R_{α} is the same as defined earlier). Unless otherwise constrained by the definition, all substituents may be further substituted by 1-3 substituents selected from hydrogen, alkyl, cycloalkyl, alkenyl, alkynyl, aryl, acyl, aralkyl, alkoxy, hydroxy, carboxy, -C(=O)OR_{ψ}, halogen, CF₃, cyano, -NR_{λ}R_{π}, -S(O)ₘR_{ψ}, -C(=O)NR_{λ}R_{π}, -OC(=O)NR_{λ}R_{π}, -CONH-, -C=O or -C=NOH (wherein R_{λ}, R_{π}, m and R_{ψ} are the same as defined earlier).

The term "alkenyl," unless otherwise specified, refers to a monoradical of a branched or unbranched unsaturated hydrocarbon group having from 2 to 20 carbon atoms with cis, trans or geminal geometry. Alkenyl groups can be optionally interrupted by atom(s) or group(s) independently chosen from oxygen, sulfur, phenylene, sulphinyl, sulphonyl and -NR_{α}- (wherein R_{α} is the same as defined earlier). In the event that alkenyl is attached to a heteroatom, the double bond cannot be alpha to the heteroatom. Alkenyl groups may be substituted further with one or more substituents selected from alkyl, alkenyl, alkynyl, alkoxy, cycloalkyl, cycloalkenyl, acyl, acylamino, acyloxy, -NHC(=O)R_{λ}, -NR_{λ}R_{π}, -C(=O)NR_{λ}R_{π}, NHC(=O)NR_{λ}R_{π}, -O-C(=O)NR_{λ}R_{π}, alkoxycarbonylamino, azido, cyano, halogen, hydroxy, oxo, keto, carboxyalkyl, thiocarbonyl, carboxy, arylthio, thiol, alkylthio, aryl, aralkyl, aryloxy, heterocyclyl, heteroaryl, heterocyclyl alkyl, heteroaryl alkyl, aminosulfonyl, aminocarbonylamino, alkoxyamino, hydroxyamino, alkoxyamino, nitro or SOₘR_{ψ} (wherein R_{λ}, R_{π}, m and R_{ψ} are as defined earlier). Unless otherwise constrained by the definition, alkenyl substituents optionally may be substituted further by 1-3 substituents selected from alkyl, alkenyl, alkynyl, carboxy, hydroxy, alkoxy, halogen, -CF₃, cyano, -NR_{λ}R_{π}, -C(=O)NR_{λ}R_{π}, -O-C(=O)NR_{λ}R_{π} and -SOₘR_{ψ} (wherein R_{λ}, R_{π}, m and R_{ψ} are as defined earlier). Groups, such as ethenyl or vinyl (CH=CH₂), 1-propylene or allyl (-CH₂CH=CH₂), iso-propylene (-C(CH₃)=CH₂), bicyclo[2.2.1]heptene, and the like, exemplify this term.

The term "alkenylene" unless otherwise specified, refers to a diradical of a branched or unbranched unsaturated hydrocarbon group preferably having from 2 to 6 carbon atoms with cis, trans or geminal geometry. In the event that alkenylene is attached to the heteroatom, the double bond cannot be alpha to the heteroatom. The alkenylene group can be connected by two bonds to the rest of the structure of compound of Formula I. Alkenylene may further be substituted with one or more substituents such as alkyl, alkenyl, alkynyl, alkoxy, cycloalkyl, acyl, acylamino, acyloxy, -NHC(=O)R_{λ}, -NR_{λ}R_{π}, -C(=O)NR_{λ}R_{π}, -NHC(=O)NR_{λ}R_{π},-OC(=O)NR_{λ}R_{π} (wherein R_{λ} and R_{π} are the same as defined earlier), alkoxycarbonylamino, azido, cyano, halogen, hydroxy, oxo, thiocarbonyl, carboxy, -COOR_{ψ} (wherein R_{ψ} is the same as defined earlier), arylthio, thiol, alkylthio, aryl, aralkyl, aryloxy, heterocyclyl, heteroaryl, heterocyclyl alkyl, heteroaryl alkyl, aminosulfonyl, alkoxyamino, nitro, -S(O)ₘR_{ψ} (wherein R_{ψ} and m are the same as defined earlier). Unless otherwise constrained by the definition, all substituents may optionally be further substituted by 1-3 substituents chosen from alkyl, alkenyl, alkynyl, carboxy, -COOR_{ψ} (wherein R_{ψ} is the same as defined earlier), hydroxy, alkoxy, halogen, -CF₃, cyano, -NR_{λ}R_{π}, -C(=O)NR_{λ}R_{π}, -OC(=O)NR_{λ}R_{π} (wherein R_{λ} and R_{π} are the same as defined earlier) and -S(O)ₘR_{ψ} (wherein R_{ψ} and m are the same as defined earlier).

The term "alkynyl," unless otherwise specified, refers to a monoradical of an unsaturated hydrocarbon, having from 2 to 20 carbon atoms. Alkynyl groups can be optionally interrupted by atom(s) or group(s) independently chosen from oxygen, sulfur, phenylene, sulphinyl, sulphonyl and -NR_{α}- (wherein R_{α} is the same as defined earlier). In the event that alkynyl groups are attached to a heteroatom, the triple bond cannot be alpha to the heteroatom. Alkynyl groups may be substituted further with one or more substituents selected from alkyl, alkenyl, alkoxy, cycloalkyl, cycloalkenyl, acyl, acylamino, acyloxy, alkoxycarbonylamino, azido, cyano, halogen, hydroxy, keto, oxo, thiocarbonyl, carboxy, carboxyalkyl, arylthio, thiol, alkylthio, aryl, aralkyl, aryloxy, aminosulfonyl, aminocarbonylamino, hydroxyamino, alkoxyamino, nitro, heterocyclyl, heteroaryl, heterocyclylalkyl, heteroarylalkyl, -NHC(=O)R_{λ}, -NR_{λ}R_{π}, -NHC(=O)NR_{λ}R_{π}, C(=O)NR_{λ}R_{π}, -O-C(=O)NR_{λ}R_{π} or -SOₘR_{ψ} (wherein R_{λ}, R_{π}, m and R_{ψ} are the same as defined earlier). Unless otherwise constrained by the definition, alkynyl substituents optionally may be substituted further by 1-3 substituents selected from alkyl, alkenyl, alkynyl, carboxy, carboxyalkyl, hydroxy, alkoxy, halogen, CF₃, -NR_{λ}R_{π}, -C(=O)NR_{λ}R_{π}, -NHC(=O)NR_{λ}R_{π}, -C(=O)NR_{λ}R_{π}, cyano or -SOₘR_{ψ} (wherein R_{λ}, R_{π}, m and R_{ψ} are the same as defined earlier).

The term "alkynylene" unless otherwise specified, refers to a diradical of a triply-unsaturated hydrocarbon, preferably having from 2 to 6 carbon atoms. In the event that alkynylene is attached to the heteroatom, the triple bond cannot be alpha to the heteroatom. The alkenylene group can be connected by two bonds to the rest of the structure of compound of Formula I. Alkynylene may further be substituted with one or more substituents such as alkyl, alkenyl, alkoxy, cycloalkyl, acyl, acylamino, acyloxy, alkoxycarbonylamino, azido, cyano, halogen, hydroxy, oxo, thiocarbonyl, carboxy, arylthio, thiol, alkylthio, aryl, aralkyl, aryloxy, aminosulfonyl, nitro, heterocyclyl, heteroaryl, heterocyclyl alkyl, heteroarylalkyl, -NHC(=O)R_{λ}, -NR_{λ}R_{π}, -NHC(=O)NR_{λ}R_{π}, -C(=O)NR_{λ}R_{π}, -OC(=O)NR_{λ}R_{π} (wherein R_{λ} and R_{π} are the same as defined earlier), -S(O)ₘR_{ψ} (wherein R_{ψ} and m are the same as defined earlier). Unless otherwise constrained by the definition, all substituents may optionally be further substituted by 1-3 substituents chosen from alkyl, alkenyl, alkynyl, carboxy, -COOR_{ψ} (wherein R_{ψ} is the same as defined earlier), hydroxy, alkoxy, halogen, CF₃, -NR_{λ}R_{π}, -C(=O)NR_{λ}R_{π}, -NHC(=O)NR_{λ}R_{π}, -C(=O)NR_{λ}R_{π} (wherein R_{λ} and R_{π} are the same as defined earlier), cyano, and -S(O)ₘR_{ψ} (wherein R_{ψ} and m are the same as defined earlier).

The term "cycloalkyl," unless otherwise specified, refers to cyclic alkyl groups of from 3 to 20 carbon atoms having a single cyclic ring or multiple condensed rings, which may optionally contain one or more olefinic bonds, unless otherwise constrained by the definition. Such cycloalkyl groups can include, for example, single ring structures, including cyclopropyl, cyclobutyl, cyclooctyl, cyclopentenyl, and the like or multiple ring structures, including adamantanyl, and bicyclo [2.2.1] heptane or cyclic alkyl groups to which is fused an aryl group, for example, indane, and the like. Spiro and fused ring structures can also be included. Cycloalkyl groups may be substituted further with one or more substituents selected from alkyl, alkenyl, alkynyl, alkoxy, cycloalkyl, cycloalkenyl, acyl, acylamino, acyloxy, alkoxycarbonylamino, azido, cyano, halogen, hydroxy, oxo, thiocarbonyl, carboxy, carboxyalkyl, arylthio, thiol, alkylthio, aryl, aralkyl, aryloxy, aminosulfonyl, aminocarbonylamino, -NR_{λ}R_{π}, -NHC(=O)NR_{λ}R_{π}, -NHC(=O)R_{λ}, -C(=O)NR_{λ}R_{π}, -O-C(=O)NR_{λ}R_{π}, nitro, heterocyclyl, heteroaryl, heterocyclylalkyl, heteroarylalkyl or SOₘR_{ψ} (wherein R_{λ}, R_{π}, m and R_{ψ} are the same as defined earlier). Unless otherwise constrained by the definition, cycloalkyl substituents optionally may be substituted further by 1-3 substituents selected from alkyl, alkenyl, alkynyl, carboxy, hydroxy, alkoxy, halogen, CF₃, -NR_{λ}R_{π}, -C(=O)NR_{λ}R_{π}, -NHC(=O)NR_{λ}R_{π},-OC(=O)NR_{λ}R_{π}, cyano or -SOₘR_{ψ} (wherein R_{λ}, R_{π}, m and R_{ψ} are the same as defined earlier). "Cycloalkylalkyl" refers to alkyl-cycloalkyl group linked through alkyl portion, wherein the alkyl and cycloalkyl are the same as defined earlier.

The term "alkoxy" denotes the group O-alkyl wherein alkyl is the same as defined above.

The term "aryl," unless otherwise specified, refers to aromatic system having 6 to 14 carbon atoms, wherein the ring system can be mono-, bi- or tricyclic and are carbocyclic aromatic groups. For example, aryl groups include, but are not limited to, phenyl, biphenyl, anthryl or naphthyl ring and the like, optionally substituted with 1 to 3 substituents selected from halogen (e.g., F, Cl, Br, I), hydroxy, alkyl, alkenyl, alkynyl, cycloalkyl, alkoxy, acyl, aryloxy, CF₃, cyano, nitro, COOR_{ψ}, NHC(=O)R_{λ}, -NR_{λ}R_{π}, -C(=O)NR_{λ}R_{π}, -NHC(=O)NR_{λ}R_{π}, -O-C(=O)NR_{λ}R_{π}, -SOₘR_{ψ}, carboxy, heterocyclyl, heteroaryl, heterocyclylalkyl, heteroarylalkyl or amino carbonyl amino, mercapto, haloalkyl, optionally substituted aryl, optionally substituted heterocyclylalkyl, thioalkyl, -CONHR_{π}, -OCOR_{π}, -COR_{π}, -NHSO₂R_{π} or -SO₂NHR_{π} (wherein R_{λ}, R_{π}, m and R_{ψ} are the same as defined earlier). Aryl groups optionally may be fused with a cycloalkyl group, wherein the cycloalkyl group may optionally contain heteroatoms selected from O, N or S. Groups such as phenyl, naphthyl, anthryl, biphenyl, and the like exemplify this term.

The term "aralkyl," unless otherwise specified, refers to alkyl-aryl linked through an alkyl portion (wherein alkyl is as defined above) and the alkyl portion contains 1-6 carbon atoms and aryl is as defined below. Examples of aralkyl groups include benzyl, ethylphenyl, propylphenyl, naphthylmethyl and the like.

The term "aralkenyl," unless otherwise specified, refers to alkenyl-aryl linked through alkenyl (wherein alkenyl is as defined above) portion and the alkenyl portion contains 1 to 6 carbon atoms and aryl is as defined below.

The term "aryloxy" denotes the group O-aryl wherein aryl is as defined above.

The term "carboxy" as defined herein refers to -C(=O)OH.

The term "heteroaryl," unless otherwise specified, refers to an aromatic ring structure containing 5 or 6 ring atoms or a bicyclic or tricyclic aromatic group having from 8 to 10 ring atoms, with one or more heteroatom(s) independently selected from N, O or S optionally substituted with 1 to 4 substituent(s) selected from halogen (e.g., F, Cl, Br, I), hydroxy, alkyl, alkenyl, alkynyl, cycloalkyl, acyl, carboxy, aryl, alkoxy, aralkyl, cyano, nitro, heterocyclyl, heteroaryl, -NR_{λ}R_{π}, CH=NOH, -(CH₂)_{w}C(=O)R_{η} {wherein w is an integer from 0-4 and R_{η} is hydrogen, hydroxy, OR_{λ}, NR_{λ}R_{π}, -NHOR_{ω} or -NHOH}, -C(=O)NR_{λ}R_{π} -NHC(=O)NR_{λ}R_{π}, -SOₘR_{ψ}, -O-C(=O)NR_{λ}R_{π}, -O-C(=O)R_{λ}, or -O-C(=O)OR_{λ} (wherein m, R_{ψ}, R_{λ} and R_{π} are as defined earlier and R_{ω} is alkyl, cycloalkyl, aryl, heteroaryl, heterocyclyl, heteroarylalkyl or heterocyclylalkyl). Unless otherwise constrained by the definition, the substituents are attached to a ring atom, *i.e.,* carbon or heteroatom in the ring. Examples of heteroaryl groups include oxazolyl, imidazolyl, pyrrolyl, 1,2,3-triazolyl, 1,2,4-triazolyl, tetrazolyl, thiazolyl, oxadiazolyl, benzoimidazolyl, thiadiazolyl, pyridinyl, pyridazinyl, pyrimidinyl, pyrazinyl, thienyl, isoxazolyl, triazinyl, furanyl, benzofuranyl, indolyl, benzthiazinyl, benzthiazinonyl, benzoxazinyl, benzoxazinonyl, quinazonyl, carbazolyl phenothiazinyl, phenoxazinyl, benzothiazolyl or benzoxazolyl, and the like.

The term "heterocyclyl," unless otherwise specified, refers to a non-aromatic monocyclic or bicyclic cycloalkyl group having 5 to 10 atoms wherein 1 to 4 carbon atoms in a ring are replaced by heteroatoms selected from O, S or N, and optionally are benzo fused or fused heteroaryl having 5-6 ring members and/or optionally are substituted, wherein the substituents are selected from halogen (e.g., F, Cl, Br, I), hydroxy, alkyl, alkenyl, alkynyl, cycloalkyl, acyl, optionally substituted aryl, alkoxy, alkaryl, cyano, nitro, oxo, carboxy, optionally substituted heterocyclyl, optionally substituted heterocyclylalkyl, optionally substituted heteroaryl, -O-C(=O)R_{λ}, -O-C(=O)OR_{λ}, -C(=O)NR_{λ}R_{π}, SOₘR_{ψ}, -O-C(=O)NR_{λ}R_{π}, -NHC(=O)NR_{λ}R_{π}, -NR_{λ}R_{π}, mercapto, haloalkyl, thioalkyl, -COOR_{ψ}, -COONHR_{λ}, -COR_{λ}, -NHSO₂R_{λ} or SO₂NHR_{λ} (wherein m, R_{ψ}, R_{λ} and R_{π} are as defined earlier) or guanidine. Heterocyclyl can optionally include rings having one or more double bonds. Such ring systems can be mono-, bi- or tricyclic. Carbonyl or sulfonyl group can replace carbon atom(s) of heterocyclyl. Unless otherwise constrained by the definition, the substituents are attached to the ring atom, *i.e.,* carbon or heteroatom in the ring. Also, unless otherwise constrained by the definition, the heterocyclyl ring optionally may contain one or more olefinic bond(s). Examples of heterocyclyl groups include oxazolidinyl, tetrahydrofuranyl, dihydrofuranyl, benzoxazinyl, benzthiazinyl, imidazolyl, benzimidazolyl, tetrazolyl, carbaxolyl, indolyl, phenoxazinyl, phenothiazinyl, dihydropyridinyl, dihydroisoxazolyl, dihydrobenzofuryl, azabicyclohexyl, thiazolidinyl, dihydroindolyl, pyridinyl, isoindole 1,3-dione, piperidinyl, tetrahydropyranyl, piperazinyl, 3H-imidazo[4,5-b]pyridine, isoquinolinyl, 1H-pyrrolo[2,3-b]pyridine or piperazinyl and the like.

"Heteroarylalkyl" refers to alkyl-heteroaryl group linked through alkyl portion, wherein the alkyl and heteroaryl are the same as defined earlier.

"Heterocyclylalkyl" refers to alkyl-heterocyclyl group linked through alkyl portion, wherein the alkyl and heterocyclyl are the same as defined earlier.

"Acyl" refers to -C(=O)R" wherein R" is selected from the group alkyl, cycloalkyl, aryl, aralkyl, heteroaryl, heterocyclyl, heteroarylalkyl or heterocyclylalkyl.

"Amine," unless otherwise specified, refers to -NH₂. "Substituted amino" unless otherwise specified, refers to a group -N(Rₖ)₂ wherein each Rₖ is independently selected from the group hydrogen provided that both Rₖ groups are not hydrogen (defined as "amino"), alkyl, alkenyl, alkynyl, aralkyl, cycloalkyl, aryl, heteroaryl, heterocyclyl, heterocyclylalkyl, heteroarylalkyl, acyl, S(O)ₘR_{ψ} (wherein m and R_{ψ} are the same as defined above), -C(=Rᵥ)NR_{λ}R_{y} (wherein Rᵥ is O or S & R_{λ} and R_{y} are the same as defined earlier) or NHC(=Rᵥ)NR_{y}R_{λ} (wherein Rᵥ, R_{y} and R_{λ} are the same as defined earlier). Unless otherwise constrained by the definition, all amino substituents may optionally be further substituted by 1-3 substituents chosen from alkyl, aralkyl, cycloalkyl, aryl, heteroaryl, heterocyclyl, carboxy, -COOR_{ψ} (wherein R_{ψ} is the same as defined earlier), hydroxy, alkoxy, halogen, CF₃, cyano, -C(=Rᵥ)NR_{λ}R_{y} (wherein Rᵥ is the same as defined earlier), -O(C=O)NR_{λ}R_{y}, -OC(=Rᵥ)NR_{λ}R_{y} (wherein R_{λ}, R_{y} and Rᵥ are the same as defined earlier), -S(O)ₘR_{ψ} (wherein R_{ψ} and m are the same as defined above).

The term "leaving group" generally refers to groups that exhibit the properties of being labile under the defined synthetic conditions and also, of being easily separated from synthetic products under defined conditions. Examples of such leaving groups include, but are not limited to, halogen (F, Cl, Br, I), triflates, tosylate, mesylates, alkoxy, thioalkoxy, hydroxy radicals and the like.

The term "activated derivative of a carboxylic acid," can include, for example, protected amino acids, aliphatic acids or aromatic acids converted to their corresponding acyl halides (e.g., acid fluoride, acid chloride and acid bromide), corresponding activated esters (e.g., nitro phenyl ester, the ester of 1-hydroxybenzotriazole or the ester of *N-*hydroxysuccinimide, HOSu), acetone oxime, bis-(4-nitrophenyl)carbonate, 2-hydroxypyridine, *N*-hydroxypthalimide, 3-nitrophenol, 4-nitrophenol, 4-nitrotrifluoroacetate, pentafluorophenol (PFP), 2,4,5-trichlorophenol, 2,2,2-trifluorophenol, 2,5-diphenyl-4-hydroxy-3-oxo-2,3-dihydrothiophene-1,1-dioxide (HOTDO), 4,6-diphenylthio[3,4-*d*]-1,3-dioxol-2-one-5,5-dioxide, *N*-ethyl-5-phenylisoxazolium-3'-sulphonate, *endo-N*-hydroxy-5-norbornene-2,3-dicarboximide (HONB)] or mixed anhydrides, for example, anhydride with ethyl chloroformate and other derivatives within the skill of the art.

The term "protecting groups" is used herein to refer to moieties which have the property of preventing specific chemical reaction at a site on the molecule undergoing chemical modification intended to be left unaffected by the particular chemical modification. Also the term protecting group, unless otherwise specified, may be used with groups such as hydroxy, amino and carboxy. Examples of such groups are found in T.W. Greene and P.G.M. Wuts, "Protective Groups in Organic Synthesis", 2nd Ed., John Wiley and Sons, New York, N.Y. The species of the carboxylic protecting groups, amino protecting groups or hydroxy protecting group employed are not critical, so long as the derivatised moieties/moiety is/are stable to conditions of subsequent reactions and can be removed without disrupting the remainder of the molecule.

"Amino acid" refers to both natural and unnatural amino acids. The term "natural amino acid," as used herein, is intended to represent the twenty two naturally-occurring amino acids glycine, alanine, valine, leucine, isoleucine, serine, methionine, threonine, phenylalanine, tyrosine, tryptophan, cysteine, proline, histidine, aspartic acid, asparagine, glutamic acid, glutamine, γ-carboxyglutamic acid, arginine, ornithine and lysine in their L form. The term "unnatural amino acid," as used herein, is intended to represent the 'D' form of the twenty two naturally-occurring amino acids described above. It is further understood that the term unnatural amino acid includes homologues of natural amino acids, and synthetically modified form of the natural amino acids commonly utilized by those in the peptide chemistry art when preparing synthetic analogues of naturally occurring peptides, including D and L forms. The synthetically modified forms include amino acids having alkylene chains shortened or lengthened by up to two carbon atoms, amino acids comprising optionally substituted aryl groups, and amino acids comprised halogenated groups preferably halogenated alkyl and aryl groups. The term "unnatural amino acids" as used herein is also intended to represent beta amino acids.

The term "peptide" refers to a molecule comprising amino acids linked through amide linkages. Dipeptide comprises of two amino acids, tripeptide refers to a peptide having three amino acids and tetrapeptide refers to one having four amino acids, wherein the term amino acid is as defined earlier. "LDVP" refers to a tetrapeptide leucyl-aspartyl-valyl-prolyl. "DVP" refers to a tripeptide aspartyl-valyl-prolyl. "VP" refers to a dipeptide valyl-prolyl.

Compounds disclosed herein contain one or more asymmetric carbon atoms and thus can exist as racemates and racemic mixtures, single enantiomers, diastereomeric mixtures and individual diastereomers. All such isomeric forms of these compounds are expressly included herein. Each stereogenic carbon may be of the R or S configuration. Although the specific compounds exemplified in this application may be depicted in a particular stereochemical configuration, compounds having either the opposite stereochemistry at any given chiral center or mixtures thereof are envisioned. Although amino acids and amino acid side chains may be depicted in a particular configuration, both natural and unnatural forms are envisioned.

### Detailed Description of the Invention

Compounds disclosed herein may be prepared by techniques well known in the art and familiar to a practitioner of ordinary skill in art. In addition, compounds disclosed herein may be prepared, by example, by the processes described herein, these processes are not the only means by which the compounds described may be synthesised. Further, synthetic steps described herein may be performed in an alternate sequence or order to give the desired compounds.

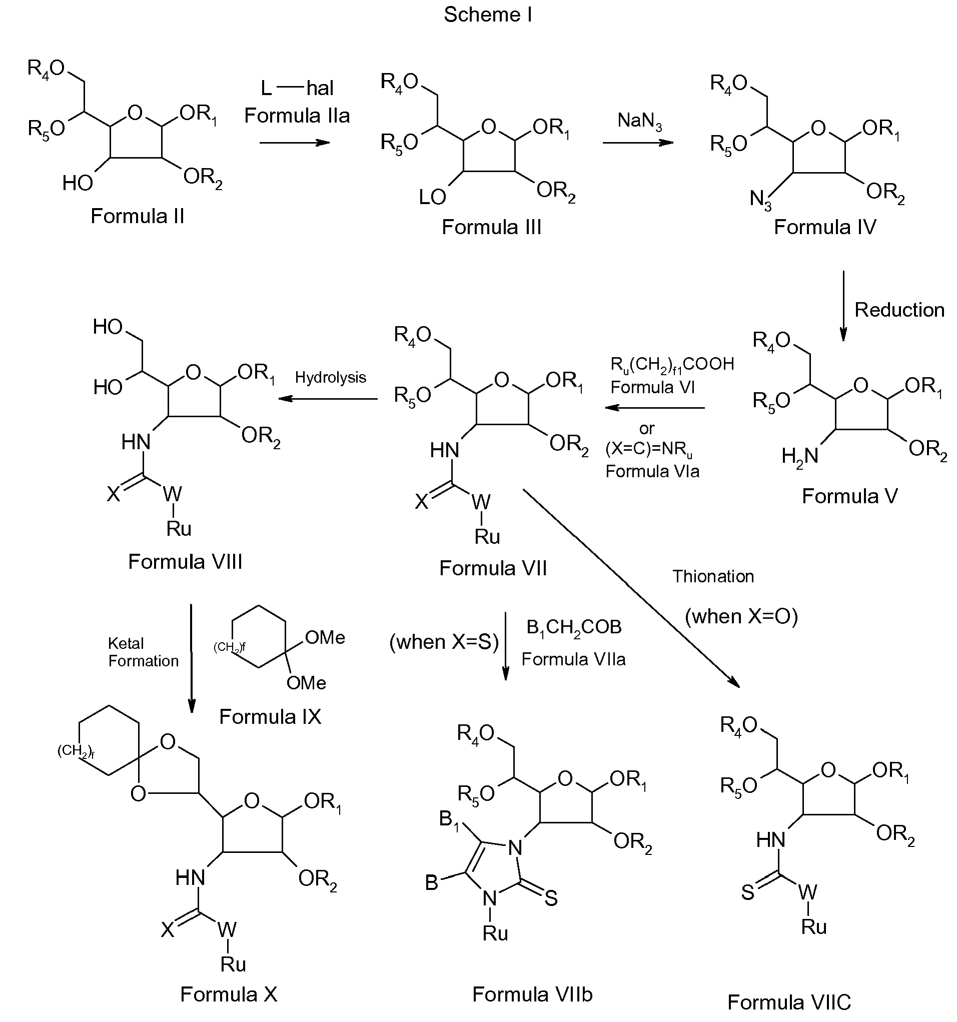

Compounds of Formula X, VIIb and VIIC can be prepared, for example, by Scheme I. Compound of Formula II (wherein R₁, R₂, R₄ and R₅ are the same as defined earlier) can be reacted with a compound of Formula IIa [wherein L is a leaving group such as tosyl, triflyl or mesyl and hal is a halogen (Cl, Br, I)] to give a compound of Formula III, which can be reacted with an zide to form a compound of Formula IV, which can undergo reduction to form a compound of Formula V, which can be reacted with a compound of Formula VI (wherein Rᵤ is selected from alkyl, alkenyl, alkynyl, cycloalkyl, aryl, aralkyl, heteroaryl, heterocyclyl, heteroarylalkyl, and heterocyclylalkyl; and when n is 0 then Y cannot be -C(=O)) and f1 is an integer from 1-3) or with a compound of Formula VIa (wherein X is oxygen or sulphur and Rᵤ is selected from alkyl, alkenyl, alkynyl, cycloalkyl, aryl, aralkyl, heteroaryl, heterocyclyl, heteroarylalkyl, and heterocyclylalkyl; and when n is 0 then Y cannot be -C(=O)) to furnish a compound of Formula VII (wherein W is -(CH₂)_{fl} or -NH and f1 is same as defined earlier), which can be hydrolyzed to give a compound of Formula VIII, which can be reacted with a compound of Formula IX (wherein f is an integer from 0-2) to give a compound of Formula X. The compound of Formula VII can be reacted with a compound of Formula VIIa (wherein B and B₁ are independently selected from the group consisting of alkyl, alkenyl, alkynyl, aryl, heteroaryl, heterocyclyl, cycloalkyl, heterocyclylalkyl or heteroarylalkyl) to give a compound of Formula VIIb. The compound of Formula VII can undergo thionation to give a compound of Formula VIIc.

The reaction of a compound of Formula II with a compound of Formula IIa to form a compound of Formula III can be carried out in the presence of an organic base, such as pyridine, trimethylamine, triethylamine, diisopropylethylamine or 2,6-lutidine.

Alternatively, the hydroxyl group in a compound of Formula II can also be converted to a triflyl group with, for example, triflic anhydride.

The reaction of a compound of Formula III with an azide to give a compound of Formula IV can be carried out in an organic solvent such as *N,N*'-dimethylformamide, dimethylsulphoxide, *N,N'*-dimethylacetamide, *N*-methylmorpholine, tetrahydrofuran, acetonitrile, dioxane or diethyl ether.

Alternatively, one may also use trimethylsilyl azide or lithium azide in place of sodium azide in the presence of catalytic amount of ammonium chloride.

The reduction of a compound of Formula IV to yield a compound of Formula V can be carried out in an organic solvent such as tetrahydrofuran, *N,N'*-dimethylformamide, diethylether or dioxane, with a reducing agent such as lithium aluminium hydride or sodium borohydride.

A compound of Formula V can be reacted with a compound of Formula VI to give a compound of Formula VII in the presence of coupling agents such as 1-(3-dimethylaminopropyl)-3-ethyl-carbodimide (EDC), *N,N'*-dicyclohexylcarbodimide (DCC), 1-cyclohexyl-3-(2-morpholinoethyl)carbodimide methyl-p-toluenesulphonate, *N,N'*-diisopropylcarbodimide (DIC), 1-(3-dimethylaminopropyl)-3-ethylcarbodimide methiodide, 2-(1-H-benzotriazol-1-yl)-1,1,3,3-tetramethyluronium hexafluorophosphate (HBTU), O-(7-azabenzotriazol-1-yl)*-N,N,N',N'*-tetramethyluronium hexafluorophosphate (HATU), (benzotriazol-1-yloxy)tris-(dimethylamino)phosphonium hexafluorophosphate (BOP), propane phosphonic acid anhydride (T3P), O-(benzotriazol-1-yl)-*N,N,N',N*'-tetramethyluronium tetrafluoroborate (TBTU), S-(1-oxido-2-pyridinyl)-1,1,3,3-tetramethylthiouronium tetrafluoroborate (TOTT), *N,N,N',N*'-tetramethyl-O-(3,4-dihydro-4-oxo-1,2,3-benzotriazin-3-yl)uronium tetrafluoroborate (TDBTU), O-(1,2-dihydro-2-oxo-pyridyl]-*N,N,N',N'*-tetramethyluronium tetrafluoroborate (TPTU), O-((ethoxycarbonyl) cyanomethylenamino)-*N,N,N',N*'-tetramethyluronium tetrafluoroborate (TOTU), 2-(5-norbornene-2,3-dicarboxamido)-1,1,3,3-tetramethyluronium tetrafluoroborate (TNTU), 2-succinimido-1,1,3,3-tetramethyluronium tetrafluroborate (TSTU), chlorotripyrrolidino phosphoniumhexafluorophosphate (PyClop), benzotriazol-1-yl-oxy-tris-pyrrolidinophosphonium hexafluorophosphate (PyBOP), chlorodipyrrolidinocarbenium hexafluorophosphate (PyClU), (benzotriazol-1-yloxy)dipiperidinocarbenium hexafluorophosphate (HBPipU) or mixtures thereof in the presence of one or more of additives or activating agents such as 1-hydroxybenzotriazole, acetone oxime, 2-hydroxypyridine, *N*-hydroxysuccinimide, pentafluorophenol (PFP), bis-(4-nitrophenyl)carbonate, *N*-hydroxypthalimide, 3-nitrophenol, 4-nitrophenol, 4-nitrotrifluoroacetate, 2,4,5-trichlorophenol, 2,2,2-trifluorophenol, 2,5-diphenyl-4-hydroxy-3-oxo-2,3-dihydrothiophene-1, 1-dioxide (HOTDO), 4,6-diphenylthio[3,4-d]-1,3-dioxol-2-one-5,5-dioxide, *N*-ethyl-5-phenylisoxazolium-3'-sulphonate, *endo-N*-hydroxy-5-norbornene-2,3-dicarboximide(HONB) or mixtures thereof and in the presence of one or more of organic bases, for example, *N*-methylmorpholine, *N*-methylmorpholine oxide, *N-*ethylmorpho line, 1-methylpiperidine, triethylamine, tribenzylamine, piperidine, *N-*ethyldiisopropylamine, 2,6-lutidine, 2,4,6-collidine, 2,4-di-tert-butyl-4-methylpyridine, 1-diethylamino-2-propanol, 1-ethylpiperidine, 1,1,3,3-tetramethylguanidine or mixtures thereof, polar aprotic solvents such as *N,N*-dimethylformamide, *N,N*-dimethylacetamide, dimethylsulphoxide, acetonitrile, 1,1,3,3,-hexafluoro-2-propanol, 1-methyl-2-pyrrolidone, tetrahydrofuran, trifluoroethanol, tetrahydrofuran, dioxane, diethyl ether or mixture thereof, halogenated solvents, for example, dichloromethane, dichloroethane, carbon tetrachloride or chloroform or mixtures thereof.

The reaction of a compound of Formula V with VIa to give a compound of Formula VII can be carried out in an organic solvent such as acetonitrile, dichloromethane, dichloroethane, chloroform or carbon tetrachloride and in the presence of one or more base for example *N*-methylmorpholine, triethylamine, *N*-methylmorpholine oxide, *N-*ethylmorpholine, 1-methylpiperidine, tribenzylamine, 2,6-lutidine or piperidine.

Alternatively, a compound of Formula VII can also be prepared by reacting a compound of Formula V with an appropriate amine in the presence of reagents such as carbonyldiimidazole (CDI) or with carbamates such as phenyl carbamate orp-nitrophenyl carbamate of an amine. Also, optionally thiocarbonyldiimidazole or an isothiocyanate can be used in place of carbonyldiimidazole or isocyanate, respectively in the reaction.

The hydrolysis of a compound of Formula VII to give a compound of Formula VIII can be carried out with the reagents, for example aqueous perchloric acid, aqueous acetic acid, aqueous sulphuric acid or Dowex 50W-8X (commercially available) in an organic solvent such as methanol, ethanol, tetrahydrofuran, *N,N*'-dimethylformamide, dioxane, acetonitrile or diethyl ether.

The reaction of a compound of Formula VIII with a compound of Formula IX to give a compound of Formula X can be carried out in an organic solvent, for example toluene, dioxane, xylene, dimethylformamide, acetonitrile o mixture(s) thereof in the presence of molecular sieves and optionally catalytic amount of the acid, for example para-toluenesulphonic acid or camphorsulphonic acid.

The reaction of a compound of Formula VII (when X = S) with a compound of Formula VIIa to give a compound of Formula VIIb can be carried out in the presence of a base, for example, triethylamine, pyridine, trimethylamine or diisopropylethylamine in an optional solvent for example, acetone, dichloromethane, dichloroethane, chloroform or carbon tetrachloride.

The thionation of a compound of Formula VII (when X = O) to give a compound of Formula VIIc can be carried out in the presence of reagents for example, phosphorous pentasulphide, Lawesson's reagent (2,4-bis-(p-methoxy)-1,3-dithiadiphosphetene-2,4-disulphide or Japanese reagent (phenyl phosphorotetrathionate) in an organic solvent for example, toluene, tetrahydrofuran, xylene, hexamethylphosphoramide (HMPA), dimethoxy ethane, benzene, dichloromethane or acetonitrile.
Particular illustrative compounds include those listed below:
1,2-O-isopropylidene-3-deoxy-3-[(3-bromobenzyl)-amido]-5,6-dioxaspiro[4,4]nonane-α-D-glucofuranoside (Compound No. 1);
1,2-O-isopropylidene-3-deoxy-3-[(3-chlorobenzyl)-amido]-5,6-dioxaspiro[4,4]nonane-α-D-glucofuranoside (Compound No. 2);
1,2-O-isopropylidene-3-deoxy-3-[(2,4-difluorobenzyl)-amido]-5,6-dioxaspiro[4,4]nonane-α-D-glucofuranoside (Compound No. 3);
1,2-O-isopropylidene-3-deoxy-3-[(2-chloro-4-fluorobenzyl)-amido]-5,6-dioxaspiro[4,4]nonane-α-D-glucofuranoside (Compound No. 4);
1,2-O-isopropylidene-3-deoxy-3-[(3-fluorobenzyl)-amido]-5,6-dioxaspiro[4,4]nonane-α-D-glucofuranoside (Compound No. 5);
1,2-O-isopropylidene-3-deoxy-3-[(3,4,5-trimethoxybenzyl)-amido]-5,6-dioxa spiro[4,4]nonane-α-D-glucofuranoside (Compound No. 6);
1,2-O-isopropylidene-3-deoxy-3-[(2,5-dimethylbenzyl)-amido]-5,6-dioxaspiro[4,4]nonane-α-D-glucofuranoside (Compound No. 7);
1,2;5,6-Di-O-isopropylidene-3-deoxy-3-(phenyl-propionamido)-α-D-glucofuranoside (Compound No. 31);
1,2;5,6-Di-O-isopropylidene-3-deoxy-3-[(2-methoxyphenyl)-propionamido]-α-D-glucofuranoside (Compound No. 32);
1,2;5,6-Di-O-isopropylidene-3-deoxy-3-[(4-fluorophenyl)-propionamido]-α-D-glucofuranoside (Compound No. 33);
1,2-O-isopropylidene-3-deoxy-3-(phenyl-urido)-α-D-glucofuranoside (Compound No 37);
1,2-O-isopropylidene-3-deoxy-3-[(4-fluorophenyl)-urido]-α-D-glucofuranoside (Compound No. 38);
1,2-O-isopropylidene-3-deoxy-3-[(2-fluorophenyl)-urido]-α-D-glucofuranoside (Compound No. 39);
1,2-O-isopropylidene-3-deoxy-3-[(4-cyanophenyl)-urido]-α-D-glucofuranoside (Compound No. 40);
1,2;5,6-Di-O-isopropylidene-3-deoxy-3-[(2-trifluoromethylphenyl)-urido]-α-D-glucofuranoside (Compound No. 41);
1,2;5,6-Di-O-isopropylidene-3-deoxy-3-[(2-methoxyphenyl)-urido]-α-D-glucofuranoside (Compound No. 42);
1,2;5,6-Di-O-isopropylidene-3-deoxy-3-[(3-fluorophenyl)-thiourido]-α-D-glucofuranoside (Compound No. 43);
1,2;5,6-Di-O-isopropylidene-3-deoxy-3-[(3-trifluoromethylphenyl)-thiourido]-α-D-glucofuranoside (Compound No. 44);
1,2-O-isopropylidene-3-deoxy-3-[(4-cyanophenyl)-thiourido]-α-D-glucofuranoside (Compound No. 45);
1,2-O-isopropylidene-3-deoxy-3-[(3-fluorophenyl)-thiourido]-α-D-glucofuranoside (Compound No. 46);
1,2-O-isopropylidene-3-deoxy-3-[(3-trifluoromethylphenyl)-thiourido]-α-D-glucofuranoside (Compound No. 47);
1,2-O-isopropylidene-3-deoxy-3-[(4-fluorophenyl)-urido]-5,6-dioxaspiro[4,5]decane-α-D-glucofuranoside (Compound No. 48);
1,2-O-isopropylidene-3-deoxy-3-[(2-fluorophenyl)-urido]-5,6-dioxaspiro[4,5]decane-α-D-glucofuranoside (Compound No. 49);
1,2-O-isopropylidene-3-deoxy-3-[(2-trifluoromethylphenyl)-urido]-5,6-dioxa spiro[4,5]decane-α-D-glucofuranoside (Compound No. 50);
1,2-O-isopropylidene-3-deoxy-3-[(4-cyanophenyl)-urido]-5,6-dioxaspiro[4,5]decane-α-D-glucofuranoside (Compound No. 51);
1,2-O-isopropylidene-3-deoxy-3-(octyl-urido)-5,6-dioxaspiro[4,5]decane-α-D-glucofuranoside (Compound No. 52);
1,2-O-isopropylidene-3-deoxy-3-(phenyl-urido)-5,6-dioxaspiro[4,5]decane-α-D-glucofuranoside (Compound No. 53);
1,2-O-isopropylidene-3-deoxy-3-[(2-methoxyphenyl)-urido]-5,6-dioxaspiro[4,5]decane-α-D-glucofuranoside (Compound No. 54);
1,2-O-isopropylidene-3-deoxy-3-[(4-trifluoromethylphenyl)-urido]-5,6-dioxa spiro[4,5]decane-α-D-glucomranoside (Compound No. 55);
1,2-O-isopropylidene-3-deoxy-3-[(3-methoxyphenyl)-thiourido]-5,6-dioxa spiro[4,5]decane-α-D-glucofuranoside (Compound No. 56);
1,2-O-isopropylidene-3-deoxy-3-[(3-fluorophenyl)-thiourido]-5,6-dioxaspiro[4,5]decane-α-D-glucofuranoside (Compound No. 57);
1,2-O-isopropylidene-3-deoxy-3-[(3-trifluoromethylphenyl)-thiourido]-5,6-dioxa spiro[4,5]decane-α-D-glucofuranoside (Compound No. 58);
1,2-O-isopropylidene-3-deoxy-3-[(3-methoxyphenyl)-urido]-5,6-dioxaspiro[4,5]decane-α-D-glucofuranoside (Compound No. 59);
1,2;5,6-Di-O-isopropylidene-3-deoxy-3-[(2-phenyl)-propionamido]-α-D-glucofuranoside (Compound No. 103);
1,2;5,6-Di-O-isopropylidene-3-deoxy-3-[3-(1,3-benzodioxol-5-yl)-propionamido]-α-D-glucofuranoside (Compound No. 104);
1,2;5,6-Di-O-isopropylidene-3-deoxy-3-(phenyl-urido)-α-D-glucofuranoside (Compound No. 107);
1,2;5,6-Di-O-isopropylidene-3-deoxy-3-[(3-bromobenzyl)-amido]-α-D-glucofuranoside (Compound No. 108);
1,2-O-isopropylidene-3-deoxy-3-[(3-bromobenzyl)-amido]-α-D-glucofuranoside (Compound No. 109);
1,2;5,6-Di-O-isopropylidene-3-deoxy-3-[3-(3-cyanophenyl)-4-methyl-2-thioxo-2,3-dihydro-1*H*-imidazol-1-yl]-α-D-glucofuranoside (Compound No. 120);
1,2;5,6-Di-O-isopropylidene-3-deoxy-3-[3-(2-nitrophenyl)-4-methyl-2-thioxo-2,3-dihydro-1*H*-imidazol-1-yl]-α-D-glucofuranoside (Compound No. 121);
1,2;5,6-Di-O-isopropylidene-3-deoxy-3-[3-(4-fluorophenyl)-4-methyl-2-thioxo-2,3-dihydro-1*H*-imidazol-1-yl]-α-D-glucofuranoside (Compound No. 122);
1,2;5,6-Di-O-isopropylidene-3-deoxy-3-[3-(2,6-dimethylphenyl)-4-methyl-2-thioxo-2,3-dihydro-1*H*-imidazol-1-yl]-α-D-glucofuranoside (Compound No. 123);
1,2;5,6-Di-O-isopropylidene-3-deoxy-3-(3-phenyl-4-methyl-2-thioxo-2,3-dihydro-1*H-*imidazol-1-yl]-α-D-glucofuranoside (Compound No. 124);
1,2;5,6-Di-O-isopropylidene-3-deoxy-3-[3-(2-methoxyphenyl)-4-methyl-2-thioxo-2,3-dihydro-1*H*-imidazol-1-yl]-α-D-glucofuranoside (Compound No. 125);
1,2;5,6-Di-O-isopropylidene-3-deoxy-3-(3-phenyl-2-thioxo-2,3-dihydro-1H-imidazo-1-yl]-α-D-glucofuranoside (Compound No. 126);
1,2;5,6-Di-O-isopropylidene-3-deoxy-3-[(3-cyanophenyl)-thiourido]-α-D-glucofuranoside (Compound No. 130);
1,2;5,6-Di-O-isopropylidene-3-deoxy-3-[(2-nitrophenyl)-thiourido]-α-D-glucofuranoside (Compound No. 131);
1,2;5,6-Di-O-isopropylidene-3-deoxy-3-[(2,6-dimethylphenyl)-thiourido]-α-D-glucofuranoside (Compound No. 132);
1,2;5,6-Di-O-isopropylidene-3-deoxy-3-[(2-methoxyphenyl)-thiourido]-α-D-glucofuranoside (Compound No. 133).
and their pharmaceutically acceptable salts, pharmaceutically acceptable solvates, esters, enantiomers, diastereomers, *N*-oxides, polymorphs, metabolites.

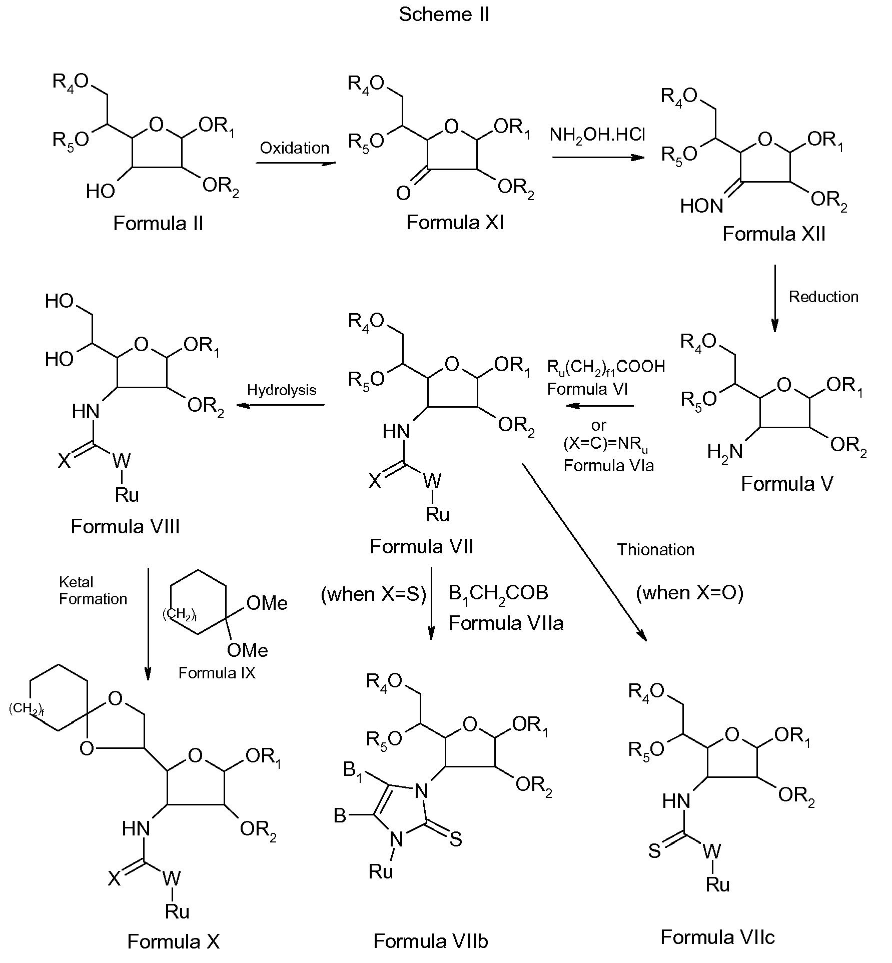

Compounds of Formula X, VIIb and VIIC can also be prepared, for example, by Scheme II. Compound of Formula II (wherein R₁, R₂, R₄ and R₅ are the same as defined earlier) can be oxidized to form a compound of Formula XI, which can be reacted with hydroxylamine hydrochloride to form a compound of Formula XII, which can undergo reduction to form a compound of Formula V, which can be reacted with a compound of Formula VI (Rᵤ is the same as defined earlier and f1 is an integer from 1-3) or with a compound of Formula VIa (wherein X and Ru are the same as defined earlier) to furnish a compound of Formula VII (wherein W is the same as defined earlier), which can be hydrolyzed to give a compound of Formula VIII, which can be reacted with a compound of Formula IX (wherein f is an integer from 0-2) to give a compound of Formula X. The compound of Formula VII can be reacted with a compound of Formula VIIa (wherein B and B₁ are independently selected from the group consisting of alkyl, alkenyl, alkynyl, aryl, heteroaryl, heterocyclyl, cycloalkyl, heterocyclylalkyl or heteroarylalkyl) to give a compound of Formula VIIb. The compound of Formula VII can undergo thionation to give a compound of Formula VIIc.

The oxidation of a compound of Formula II to form a compound of Formula XI can be carried out under various conditions. For example, one may use Swern's oxidation utilizing dimethyl sulphoxide and acetic anhydride or oxalyl chloride, optionally in either dimethyl sulphoxide or dichloromethane as solvents. One may also utilize oxidizing agents such as pyridinium chlorochromate, pyridinium dichromate, pyridine-sulfurtrioxide or Dess-Martin periodinane in an organic solvent such as dichloromethane or chloroform.

Thus, the oxidation of a compound of Formula II can be carried out, for example, utilizing dimethyl sulphoxide and acetic anhydride to furnish a compound of Formula XI.

The reaction of a compound of Formula XI with hydroxylamine hydrochloride to form a compound of Formula XII can be carried out in an organic solvent such as ethanol, methanol, propanol or isopropyl alcohol, in the presence of an organic base such as pyridine, triethylamine or diisopropylethylamine.

The reduction of a compound of Formula XII to yield a compound of Formula V can be carried out in an organic solvent such as tetrahydrofuran, *N,N'-*dimethylformamide, diethylether or dioxane, with a reducing agent such as lithium aluminium hydride or sodium borohydride.

A compound of Formula V can be reacted with a compound of Formula VI to give a compound of Formula VII in the presence of coupling agents such as 1-(3-dimethylaminopropyl)-3-ethyl-carbodimide (EDC), *N*,*N*'-dicyclohexylcarbodimide (DCC), 1-cyclohexyl-3-(2-morpholinoethyl)carbodimide methyl-p-toluenesulphonate, N, *N*'-diisopropylcarbodimide (DIC), 1-(3-dimethylaminopropyl)-3-ethylcarbodimide methiodide, 2-(1-H-benzotriazol-1-yl)-1,1,3,3-tetramethyluronium hexafluorophosphate (HBTU), O-(7-azabenzotriazol-1-yl)-*N,N,N',N*'-tetramethyluronium hexafluorophosphate (HATU), (benzotriazol-1-yloxy)tris-(dimethylamino)phosphonium hexafluorophosphate (BOP), propane phosphonic acid anhydride (T3P), O-(benzotriazol-1-yl)-*N*,*N*,*N'*,*N*'-tetramethyluronium tetrafluoroborate (TBTU), S-(1-oxido-2-pyridinyl)-1,1,3,3-tetramethylthiouronium tetrafluoroborate (TOTT), *N*,*N*,*N*',*N*'-tetramethyl-O-(3,4-dihydro-4-oxo-1,2,3-benzotriazin-3-yl)uronium tetrafluoroborate (TDBTU), O-(1,2-dihydro-2-oxo-pyridyl]-*N*,*N*,*N*',*N*'-tetramethyluronium tetrafluoroborate (TPTU), O-((ethoxycarbonyl) cyanomethylenamino)-*N,N,N',N*'-tetramethyluronium tetrafluoroborate (TOTU), 2-(5-norbornene-2,3-dicarboxamido)-1,1,3,3-tetramethyluronium tetrafluoroborate (TNTU), 2-succinimido-1,1,3,3-tetramethyluronium tetrafluroborate (TSTU), chlorotripyrrolidino phosphoniumhexafluorophosphate (PyClop), benzotriazol-1-yl-oxy-tris-pyrrolidinophosphonium hexafluorophosphate (PyBOP), chlorodipyrrolidinocarbenium hexafluorophosphate (PyClU), (benzotriazol-1-yloxy)dipiperidinocarbenium hexafluorophosphate (HBPipU) or mixtures thereof in the presence of one or more of additives or activating agents such as 1-hydroxybenzotriazole, acetone oxime, 2-hydroxypyridine, *N*-hydroxysuccinimide, pentafluorophenol (PFP), bis-(4-nitrophenyl)carbonate, *N*-hydroxypthalimide, 3-nitrophenol, 4-nitrophenol, 4-nitrotrifluoroacetate, 2,4,5-trichlorophenol, 2,2,2-trifluorophenol, 2,5-diphenyl-4-hydroxy-3-oxo-2,3-dihydrothiophene-1, 1-dioxide (HOTDO), 4,6-diphenylthio[3,4-d]-1,3-dioxol-2-one-5,5-dioxide, *N*-ethyl-5-phenylisoxazolium-3'-sulphonate, *endo-N*-hydroxy-5-norbornene-2,3-dicarboximide (HONB) or mixtures thereof and in the presence of one or more of organic bases, for example, *N*-methylmorpholine, *N*-methylmorpholine oxide, *N-*ethylmorpho line, 1-methylpiperidine, triethylamine, tribenzylamine, piperidine, *N-*ethyldiisopropylamine, 2,6-lutidine, 2,4,6-collidine, 2,4-di-tert-butyl-4-methylpyridine, 1-diethylamino-2-propanol, 1-ethylpiperidine, 1,1,3,3-tetramethylguanidine or mixtures thereof, polar aprotic solvents such as *N,N*-dimethylformamide, *N,N*-dimethylacetamide, dimethylsulphoxide, acetonitrile, 1,1,3,3,-hexafluoro-2-propanol, 1-methyl-2-pyrrolidone, tetrahydrofuran, trifluoroethanol, tetrahydrofuran, dioxane, diethyl ether or mixture thereof, halogenated solvents, for example, dichloromethane, dichloroethane, carbon tetrachloride or chloroform or mixtures thereof.

The reaction of a compound of Formula V with VIa to give a compound of Formula VII can be carried out in an organic solvent such as acetonitrile, dichloromethane, dichloroethane, chloroform or carbon tetrachloride and in the presence of one or more base for example *N*-methylmorpholine, triethylamine, *N*-methylmorpholine oxide, *N-*ethylmorpholine, 1-methylpiperidine, tribenzylamine, 2,6-lutidine or piperidine.

Alternatively, a compound of Formula VII can also be prepared by reacting a compound of Formula V with an appropriate amine in the presence of reagents such as carbonyldiimidazole (CDI) or with carbamates such as phenyl carbamate or p-nitrophenyl carbamate of an amine. Also, optionally thiocarbonyldiimidazole or an isothiocyanate can be used in place of carbonyldiimidazole or isocyanate, respectively in the reaction.

The hydrolysis of a compound of Formula VII to give a compound of Formula VIII can be carried out with the reagents, for example aqueous perchloric acid, aqueous acetic acid, aqueous sulphuric acid or Dowex 50W-8X (commercially available) in an organic solvent such as methanol, ethanol, tetrahydrofuran, *N*,*N*'-dimethylformamide, dioxane or diethyl ether.

The reaction of a compound of Formula VIII with a compound of Formula IX to give a compound of Formula X can be carried out in an organic solvent, for example toluene, dioxane, xylene, dimethylformamide, acetonitrile o mixture(s) thereof in the presence of molecular sieves and optionally catalytic amount of the acid, for example para-toluenesulphonic acid or camphorsulphonic acid.

The reaction of a compound of Formula VII (when X =S) with a compound of Formula VIIa to give a compound of Formula VIIb can be carried out in the presence of a base, for example, triethylamine, pyridine, trimethylamine or diisopropylethylamine in an optional solvent for example, acetone, dichloromethane, dichloroethane, chloroform or carbon tetrachloride.

The thionation of a compound of Formula VII (when X =O)to give a compound of Formula VIIc can be carried out in the presence of reagents for example, phosphorous pentasulphide, Lawesson's reagent (2,4-bis-(p-methoxy)-1,3-dithiadiphosphetene-2,4-disulphide or Japanese reagent (phenyl phosphorotetrathionate) in an organic solvent for example, toluene, tetrahydrofuran, xylene, hexamethylphosphoramide (HMPA), dimethoxy ethane, benzene, dichloromethane or acetonitrile.
Particular illustrative compounds which may be prepared following, for example, Scheme II include:
1,2-O-isopropylidene-3-deoxy-3-[(3-fluorobenzyl)-amido]-5,6-dioxaspiro[4,4]nonane-α-D-allofuranoside (Compound No. 8);
1,2-O-isopropylidene-3-deoxy-3-[(4-fluorobenzyl)-amido]-5,6-dioxaspiro[4,4]nonane-α-D-allofuranoside (Compound No. 9);
1,2-O-isopropylidene-3-deoxy-3-[(2-chloro-4-fluorobenzyl)-amido]-5,6-dioxa spiro[4,4]nonane-α-D-allofuranoside (Compound No. 10);
1,2-O-isopropylidene-3-deoxy-3-[(3-bromobenzyl)-amido]-5,6-dioxaspiro[4,4]nonane-α-D-allofuranoside (Compound No. 11);
1,2-O-isopropylidene-3-deoxy-3-[(3-chlorobenzyl)-amido]-5,6-dioxaspiro[4,4]nonane-α-D-allofuranoside (Compound No. 12);
1,2-O-isopropylidene-3-deoxy-3-[(2,5-dimethylbenzyl)-amido]-5,6-dioxaspiro[4,4]nonane-α-D-allofuranoside (Compound No. 13);
1,2-O-isopropylidene-3-deoxy-3-[(3,4,5-trimethoxybenzyl)-amido]-5,6-dioxa spiro[4,4]nonane-α-D-allofuranoside (Compound No. 14);
1,2;5,6-Di-O-isopropylidene-3-deoxy-3-(phenyl-propionamido)-α-D-allofuranoside (Compound No. 34);
1,2;5,6-Di-O-isopropylidene-3-deoxy-3-[(2-methoxyphenyl)-propionamido]-α-D-allofuranoside (Compound No. 35);
1,2;5,6-Di-O-isopropylidene-3-deoxy-3-[(4-fluorophenyl)-propionamido]-α-D-allofuranoside (Compound No. 36);
1,2-O-isopropylidene-3-deoxy-3-[4-fluorophenyl)-urido]-α-D-allofuranoside (Compound No. 60);
1,2-O-isopropylidene-3-deoxy-3-[(2-fluorophenyl)-urido]-α-D-allofuranoside (Compound No. 61);
1,2;5,6-Di-O-isopropylidene-3-deoxy-3-[(4-trifluoromethylphenyl)-urido]-a-D-allofuranoside (Compound No. 62);
1,2;5,6-Di-O-isopropylidene-3-deoxy-3-(phenyl-urido)-α-D-allofuranoside (Compound No. 63);
1,2;5,6-Di-O-isopropylidene-3-deoxy-3-[(2-methoxyphenyl)-urido]-α-D-allofuranoside (Compound No. 64);
1,2;5,6-Di-O-isopropylidene-3-deoxy-3-[4-cyanophenyl)-urido]-α-D-allofuranoside (Compound No. 65);
1,2-O-isopropylidene-3-deoxy-3-[(4-trifluoromethylphenyl)-urido]-α-D-allofuranoside (Compound No. 66);
1,2-O-isopropylidene-3-deoxy-3-(phenyl-urido)-α-D-allofuranoside (Compound No. 67);
1,2-O-isopropylidene-3-deoxy-3-[(2-methoxyphenyl)-urido]-α-D-allofuranoside (Compound No. 68);
1,2-O-isopropylidene-3-deoxy-3-[(4-cyanophenyl)-urido]-α-D-allofuranoside (Compound No. 69);
1,2-O-isopropylidene-3-deoxy-3-[(2-trifluoromethylphenyl)-urido]-oc-D-allofuranoside (Compound No. 70);
1,2-O-isopropylidene-3-deoxy-3-[(4-fluorophenyl)-thiourido]-α-D-allofuranoside (Compound No. 71);
1,2-O-isopropylidene-3-deoxy-3-[(2-fluorophenyl)-thiourido]-α-D-allofuranoside (Compound No. 72);
1,2-O-isopropylidene-3-deoxy-3-[(2-trifluoromethylphenyl)-thiourido]-α-D-allofuranoside (Compound No. 73);
1,2-O-isopropylidene-3-deoxy-3-[(2-methoxyphenyl)-thiourido]-α-D-allofuranoside (Compound No. 74);
1,2-O-isopropylidene-3-deoxy-3-(phenyl-thiourido)-α-D-allofuranoside (Compound No. 75);
1,2-O-isopropylidene-3-deoxy-3-[(4-trifluoromethylphenyl)-thiourido]-α-D-allofuranoside (Compound No. 76);
1,2-O-isopropylidene-3-deoxy-3-[(4-cyanophenyl)-thiourido]-α-D-allofuranoside (Compound No. 77);
1,2-O-isopropylidene-3-deoxy-3-[(4-fluorophenyl)-thiourido]-5,6-dioxa spiro[4,5]decane-α-D-allofuranoside (Compound No. 78);
1,2-O-isopropylidene-3-deoxy-3-[(2-fluorophenyl)-thiourido]-5,6-dioxa spiro[4,5]decane-α-D-allofuranoside (Compound No. 79);
1,2-O-isopropylidene-3-deoxy-3-[(2-trifluoromethylphenyl)-thiourido]-5,6-dioxa spiro[4,5]decane-α-D-allofuranoside (Compound No. 80);
1,2-O-isopropylidene-3-deoxy-3-(phenyl-thiourido)-5,6-dioxa spiro[4,5]decane-α-D-allofuranoside (Compound No. 81);
1,2-O-isopropylidene-3-deoxy-3-[(4-trifluoromethylphenyl)-thiourido]-5,6-dioxa spiro[4,5]decane-α-D-allofuranoside (Compound No. 82);
1,2-O-isopropylidene-3-deoxy-3-[(4-cyanophenyl)-thiourido]-5,6-dioxaspiro[4,5]decane-α-D-allofuranoside (Compound No. 83);
1,2-O-isopropylidene-3-deoxy-3-[(2-methoxyphenyl)-urido]-5,6-dioxaspiro[4,5]decane-α-D-allofuranoside (Compound No. 84);
1,2;5,6-Di-O-isopropylidene-3-deoxy-3-[(3-fluorophenyl)-urido]-α-D-allofuranoside (Compound No. 85);
1,2-O-isopropylidene-3-deoxy-3-[(3-fluorophenyl)-urido]-α-D-allofuranoside (Compound No. 86);
1,2-O-isopropylidene-3-deoxy-3-[(3-cyanophenyl)-urido]-α-D-allofuranoside (Compound No. 87);
1,2;5,6-Di-O-isopropylidene-3-deoxy-3-[(3-fluorophenyl)-thiourido]-α-D-allofuranoside (Compound No. 88);
1,2;5,6-Di-O-isopropylidene-3-deoxy-3-[3-trifluoromethylphenyl)-thiourido]-α-D-allofuranoside (Compound No. 89);
1,2-O-isopropylidene-3-deoxy-3-[(4-trifluoromethylphenyl)-urido]-5,6-dioxa spiro[4,5]decane-a-D-allofuranoside (Compound No. 90);
1,2-O-isopropylidene-3-deoxy-3-[(2-trifluoromethylphenyl)-urido]-5,6-dioxa spiro[4,5]decane- -D-allofuranoside (Compound No. 91);
1,2-O-isopropylidene-3-deoxy-3-[(4-cyanophenyl)-urido]-5,6-dioxaspiro[4,5]decane-α-D-allofuranoside (Compound No. 92);
1,2-O-isopropylidene-3-deoxy-3-[(3-fluorophenyl)-thiourido]-α-D-allofuranoside (Compound No. 93);
1,2-O-isopropylidene-3-deoxy-3-[(3-trifluoromethylphenyl)-thiourido]-α-D-allofuranoside (Compound No. 94);
1,2-O-isopropylidene-3-deoxy-3-[(3-methoxyphenyl)-thiourido]-α-D-allofuranoside (Compound No. 95);
1,2-O-isopropylidene-3-deoxy-3-[(4-fluorophenyl)-urido]-5,6-dioxaspiro[4,5]decane-α-D-allofuranoside (Compound No. 96);
1,2-O-isopropylidene-3-deoxy-3-[(2-fluorophenyl)-urido]-5,6-dioxaspiro[4,5]decane-α-D-allofuranoside (Compound No. 97);
1,2-O-isopropylidene-3-deoxy-3-[(3-fluorophenyl)-urido]-5,6-dioxaspiro[4,5]decane-α-D-allofuranoside (Compound No. 98);
1,2-O-isopropylidene-3-deoxy-3-[(3-cyanophenyl)-urido]-5,6-dioxaspiro[4,5]decane-α-D-allofuranoside (Compound No. 99);
1,2-O-isopropylidene-3-deoxy-3-(phenyl-urido)-5,6-dioxaspiro[4,5]decane-α-D-allofuranoside (Compound No. 100);
1,2;5,6-Di-O-isopropylidene-3-deoxy-3-[(3-methoxyphenyl)-urido]-α-D-allofuranoside (Compound No. 101);
1,2-O-isopropylidene-3-deoxy-3-[(3-methoxyphenyl)-urido]-α-D-allofuranoside (Compound No. 102);
1,2;5,6-Di-O-isopropylidene-3-deoxy-3-[(2-phenyl)propionamido]-α-D-allofuranoside (Compound No. 105);
1,2;5,6-Di-O-isopropylidene-3-deoxy-3-[3-(1,3-benzodioxol-5-yl)-propionamido]-α-D-allofuranoside (Compound No. 106);
1,2;5,6-Di-O-isopropylidene-3-deoxy-3-[(4-fluorophenyl)-urido]-α-D-allofuranoside (Compound No. 110);
1,2;5,6-Di-O-isopropylidene-3-deoxy-3-[(3-fluorobenzyl)-amido]-α-D-allofuranoside (Compound No. 111);
1,2-O-isopropylidene-3-deoxy-3-[(3-fluorobenzyl)-amido]-α-D-allofuranoside (Compound No. 112);
1,2;5,6-Di-O-isopropylidene-3-deoxy-3-[3-(4-cyanophenyl)-4-methyl-2-thioxo-2,3-dihydro-1*H*-imidazol-1-yl]-α-D-allofuranoside (Compound No. 113);
1,2;5,6-Di-O-isopropylidene-3-deoxy-3-[3-(2-fluorophenyl)-4-methyl-2-thioxo-2,3-dihydro-1*H*-imidazol-1-yl]-α-D-allofuranoside (Compound No. 114);
1,2;5,6-Di-O-isopropylidene-3-deoxy-3-[3-(2-methoxyphenyl)-4-methyl-2-thioxo-2,3-dihydro-1*H*-imidazol-1-yl]-α-D-allofuranoside (Compound No. 115);
1,2;5,6-Di-O-isopropylidene-3-deoxy-3-[3-(2,6-dimethylphenyl)-4-methyl-2-thioxo-2,3-dihydro-1*H*-imidazol-1-yl]-α-D-allofuranoside (Compound No. 116);
1,2;5,6-Di-O-isopropylidene-3-deoxy-3-[3-(3-methoxyphenyl)-4-methyl-2-thioxo-2,3-dihydro-1*H*-imidazol-1-yl]-α-D-allofuranoside (Compound No. 117);
1,2;5,6-Di-O-isopropylidene-3-deoxy-3-(3-phenyl-4-methyl-2-thioxo-2,3-dihydro-1*H-*imidazol-1-yl]-α-D-allofuranoside (Compound No. 118);
1,2;5,6-Di-O-isopropylidene-3-deoxy-3-[3-(2-trifluoromethylphenyl)-4-methyl-2-thioxo-2,3-dihydro-1*H*-imidazol-1-yl]-α-D-allo furanoside (Compound No. 119);
1,2;5,6-Di-O-isopropylidene-3-deoxy-3-[(4-fluorobenzyl)-thioamido]-α-D-allo furanoside (Compound No. 127);
1,2;5,6-Di-O-isopropylidene-3-deoxy-3-[(2-chloro-4-fluorobenzyl)-thioamido]-α-D-allofuranoside (Compound No. 128);
1,2;5,6-Di-O-isopropylidene-3-deoxy-3-[(2-methoxybenzyl)-thioamido]-α-D-allofuranoside (Compound No. 129);
1,2;5,6-Di-O-isopropylidene-3-deoxy-3-[3-(4-fluorohenyl)-4-methyl-2-thioxo-2,3-dihydro-1*H*-imidazol-1-yl]-α-D-allofuranoside (Compound No. 134);
and their pharmaceutically acceptable salts, pharmaceutically acceptable solvates, esters, enantiomers, diastereomers, *N*-oxides, polymorphs, metabolites.

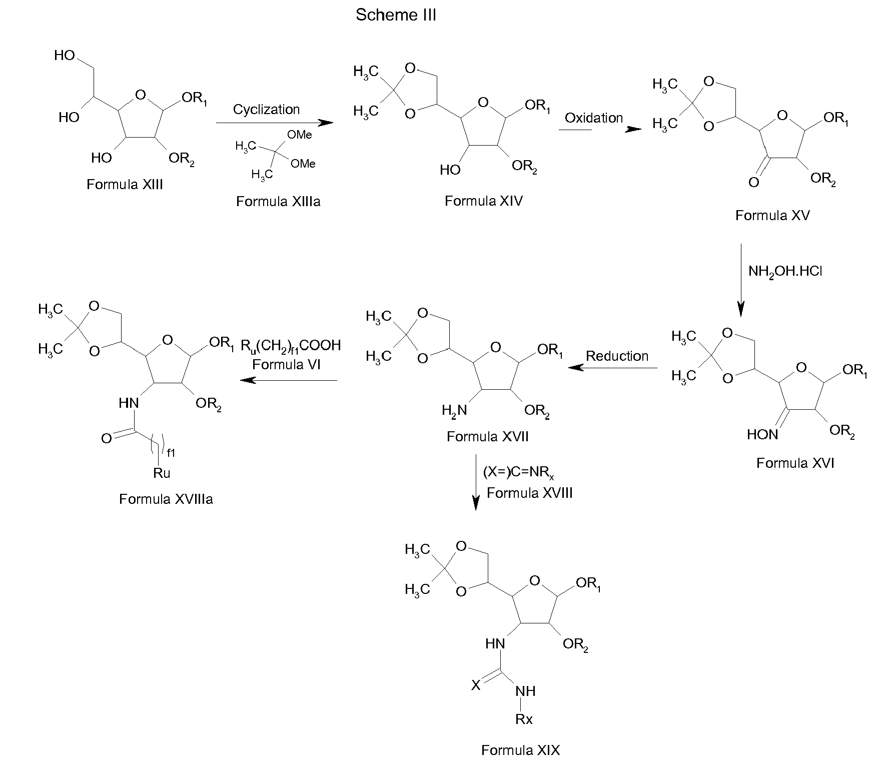

The compounds of Formula XVIIIa and XIX can be prepared, for example, by following the procedure as described in Scheme III. Thus the compound of Formula XIII (wherein R₁ and R₂ are the same as defined earlier) undergoes cyclization with a compound of Formula XIIIa to give a compound of Formula XIV, which can be oxidized to give a compound of Formula XV, which can be reacted with hydroxyl amine hydrochloride to give a compound of Formula XVI, which can be reduced to give a compound of Formula XVII, which can be reacted with a compound of Formula VI (where Rᵤ and fl are same as defined earlier) to give a compound of Formula XVIIIa. The compound of Formula XVII can be reacted with a compound of Formula XVIII (wherein X and Rₓ is the same as defined earlier) to give a compound of Formula XIX.

The cyclization of a compound of Formula XIII with a compound of Formula XIIIa to give a compound of Formula XIV can be carried out in an organic solvent such as xylene, toluene or acetonitrile in the presence of an acid such as, para-toluenesulphonic acid, camphor sulphonic acid or formic acid.

Oxidation of a compound of Formula XIV to give a compound of Formula XV can be carried out under various conditions. For example, one may use Swern's oxidation utilizing dimethyl sulphoxide and acetic anhydride or oxalyl chloride, optionally in either dimethyl sulphoxide or dichloromethane as solvents. One may also utilize oxidizing agents such as pyridinium chlorochromate, pyridinium dichromate, pyridine-sulfurtrioxide or Dess-Martin periodinane in an organic solvent such as dichloromethane, carbon tetrachloride, chloroform or dichloroethane.

Thus, the oxidation of a compound of Formula XIV can be carried out utilizing dimethyl sulphoxide and acetic anhydride to furnish a compound of Formula XV.

The reaction of a compound of Formula XV with hydroxylamine hydrochloride to form a compound of Formula XVI can be carried out in an organic solvent such as ethanol, methanol, propanol or isopropyl alcohol, in the presence of an organic base such as pyridine, trimethylamine, triethylamine, diisopropylethylamine or 2,6-lutidine.

Alternatively inorganic bases may be used in place of organic bases such as, potassium carbonate, sodium carbonate or sodium bicarbonate in an organic solvent such as methanol, ethanol, propanol or isopropylalcohol or mixture thereof.

The reduction of a compound of Formula XVI to yield a compound of Formula XVII can be carried out in an organic solvent such as tetrahydrofuran, dimethylformamide, diethylether or dioxane, with a reducing agent such as lithium aluminium hydride or sodium borohydride.

A compound of Formula XVII can be reacted with a compound of Formula VI to give a compound of Formula XVIIIa in the presence of coupling agents such as 1-(3-dimethylaminopropyl)-3-ethyl-carbodiimide, *N,N*'-dicyclohexylcarbodiimide, 2-(1-H-benzotriazol-1-yl)-1,1,3,3-tetramethyluronium hexafluorophosphate (HBTU), O-(7-azabenzotriazol-1-yl)-*N,N,N',N*'-tetramethyluronium hexafluorophosphate (HATU), (benzotriazol-1-yloxy)tris-(dimethylamino)phosphonium hexafluorophosphate (BOP), propane phosphonic acid anhydride (T3P), O-(benzotriazol-1-yl)-*N*,*N*,*N*',*N*'-tetramethyluronium tetrafluoroborate (TBTU), S-(1-oxido-2-pyridinyl)-1,1,3,3-tetramethylthiouronium tetrafluoroborate (TOTT), *N,N,N',N*'-tetramethyl-O-(3,4-dihydro-4-oxo-1,2,3-benzotriazin-3-yl)uronium tetrafluoroborate (TDBTU), O-(1,2-dihydro-2-oxo-pyridyl]-*N,N,N',N'*-tetramethyluronium tetrafluoroborate (TPTU), O-((ethoxycarbonyl) cyanomethylenamino)-*N,N,N',N*'-tetramethyluronium tetrafluoroborate (TOTU), chlorotripyrrolidino phosphoniumhexafluorophosphate (PyClop), benzotriazol-1-yl-oxy-tris-pyrrolidino-phosphonium hexafluorophosphate (PyBOP), chlorodipyrrolidinocarbenium hexafluorophosphate (PyClU), (benzotriazol-1-yloxy)dipiperidinocarbenium hexafluorophosphate (HBPipU) or mixtures thereof in the presence of one or more of additives or activating agents such as 1-hydroxybenzotriazole, acetone oxime, 2-hydroxypyridine, *N*-hydroxysucciniimide, pentafluorophenol or mixtures thereof and in the presence of one or more of organic bases, for example, *N-*methylmorpho line, *N*-methylmorpholine oxide, *N*-ethylmorpholine, 1-methylpiperidine, triethylamine, tribenzylamine, piperidine, *N*-ethyldiisopropylamine, 2,6-lutidine or mixtures thereof, polar aprotic solvents such as dimethylformamide or dimethylsulphoxide, ethers, for example, tetrahydrofuran, dioxane or diethyl ether, halogenated solvents, for example, dichloromethane, dichloroethane, carbon tetrachloride or chloroform or mixtures thereof.

The compound of Formula XVII can be reacted with a compound of Formula XVIII to give a compound of Formula XIX in an organic solvent such as acetonitrile, dichloromethane, dichloroethane, chloroform or carbon tetrachloride and one or more bases *N*-methylmorpholine, triethylamine *N*-methylmorpholine oxide, *N*-ethylmorpholine, 1-methylpiperidine, tribenzylamine, or piperidine.

Alternatively, a compound of Formula XIX can also be prepared by reacting a compound of Formula XVII with an appropriate amine in the presence of reagents such as carbonyldiimidazole (CDI) or with carbamates such as phenyl carbamate or *p*-nitrophenyl carbamate of an amine. Also, optionally thiocarbonyldiimidazole or an isothiocyanate can be used in place of carbonyldiimidazole or isocyanate, respectively in the reaction.
Particular illustrative compounds include those listed below:
1,2-Dioxaspiro[2,5]octane-3-deoxy-3-[(4-fluorophenyl)-urido]-5,6-O-isopropylidene-α-D-allofuranoside (Compound No. 15);
1,2-Dioxaspiro[2,5]octane-3-deoxy-3-(phenyl-urido)-5,6-O-isopropylidene-α-D-allofuranoside (Compound No. 16);
1,2-Dioxaspiro[2,5]octane-3-deoxy-3-[(4-cyanophenyl)-urido]-5,6-O-isopropylideneα-D-allofuranoside (Compound No. 17);
1,2-Dioxa spiro[2,5]octane-3-deoxy-3-[(4-trifluoromethylphenyl)-urido]-5,6-O-isopropylidene-α-D-allofuranoside (Compound No. 18);
1,2-Dioxa spiro[2,5]octane-3-deoxy-3-[(2-trifluoromethylphenyl)-urido]-5,6-O-isopropylidene-α-D-allofuranoside (Compound No. 19);
1,2-Dioxa spiro[2,5]octane-3-deoxy-3-[(4-fluorophenyl)-thiourido]-5,6-O-isopropylidene-α-D-allofuranoside (Compound No. 20);
1,2-Dioxa spiro[2,5]octane-3-deoxy-3-(phenyl-thiourido)-5,6-O-isopropylidene-α-D-allofuranoside (Compound No. 21);
1,2-Dioxa spiro[2,5]octane-3-deoxy-3-[(4-cyanophenyl)-thiourido]-5,6-O-isopropylidene-α-D-allofuranoside (Compound No. 22);
1,2-Dioxa spiro[2,5]octane-3-deoxy-3-[(4-trifluoromethylphenyl)-thiourido]-5,6-O-isopropylidene-α-D-allofuranoside (Compound No. 23);
1,2-Dioxa spiro[2,5]octane-3-deoxy-3-[(2-trifluoromethylphenyl)-thiourido]-5,6-O-isopropylidene-α-D-allofuranoside (Compound No. 24);
1,2-Dioxa spiro[2,5]octane-3-deoxy-3-[(3-chlorobenzyl)-amido]-5,6-O-isopropylidene-α-D-allofuranoside (Compound No. 25);
1,2-Dioxa spiro[2,5]octane-3-deoxy-3-[(3-fluorobenzyl)-amido]-5,6-O-isopropylidene-α-D-allofuranoside (Compound No. 26);
1,2-Dioxa spiro[2,5]octane-3-deoxy-3-[(4-fluorobenzyl)-amido]-5,6-O-isopropylidene-α-D-allofuranoside (Compound No. 27);
1,2-Dioxa spiro[2,5]octane-3-deoxy-3-[(2-chloro-4-fluorobenzyl)-amido]-5,6-O-isopropylidene-α-D-allofuranoside (Compound No. 28);
1,2-Dioxa spiro[2,5]octane-3-deoxy-3-[(2,5-dimethylbenzyl)-amido]-5,6-O-isopropylidene-α-D-allofuranoside (Compound No. 29);
1,2-Dioxa spiro[2,5]octane-3-deoxy-3-[(3,4,5-trimethoxybenzyl)-amido]-5,6-O-isopropylidene-α-D-allofuranoside (Compound No. 30);
and their pharmaceutically acceptable salts, pharmaceutically acceptable solvates, esters, en antiomers, diastereomers, *N*-oxides, polymorphs, metabolites.

Also, in all the above representative examples wherever esters are specified, one skilled in the art could optionally hydrolyze them to their respective acids, for example hydrolysis of alkyl esters (such as ethyl, methyl or benzyl ester) to their corresponding acids can be carried out in the presence of a base, for example, lithium hydroxide, sodium hydroxide or potassium hydroxide. Alternatively, hydrolysis of benzyl ester can be carried out hydrogenatically using catalysts, for example, palladium on carbon or platinum on carbon. Esters such as tert-butyl can be hydrolyzed to their corresponding acids in the presence of acid, for example, trifluoroacetic acid or hydrochloric acid.

In the above schemes, where specific bases, acids, solvents, condensing agents, hydrolyzing agents, etc., are mentioned, it is to be understood that other acids, bases, solvents, condensing agents, hydrolyzing agents, etc., may also be used. Similarly, the reaction temperature and duration of the reactions may be adjusted according to the requirements that arise during the process.

While the present invention has been described in terms of its specific embodiment, certain modification and equivalent will be apparent to those skilled in the art and are included within the scope of the present invention. The examples are provided to illustrate particular aspects of the disclosure and do not limit the scope of the present invention.

### EXAMPLES

### General Synthesis:

### Synthesis of 1,2;5,6-Di-O-isopropylidene-3-deoxy-3-amino-α-D-allofuranoside

### Step a: 1,2;5,6-Di-O-isopropylidene-3-oxo-α-D-glucofuranoside

To a solution of 1,2;5,6-Di-O-isopropylidene-α-D-glucofuranoside (10 g, 38.41 mmol) in dry dimethyl sulphoxide (60 ml) added acetic anhydride (20 ml, 211.76 mmol). The reaction mixture was stirred at 60-70°C for 5-6 hours. After the completion of the reaction, cooled to 0 °C and added water (15 ml) to it with vigorous stirring till the colour of the reaction mixture became light yellow. Extracted the reaction mixture with ethyl acetate, washed with water, dried over anhydrous sodium sulphate, filtered and evaporated the solvent under reduced pressure to afford the title compound as dark orange brown oil. Yield : 13 g.

### Step b: 1,2;5,6-Di-O-isopropylidene-3-deoxy-3-hydroxyimino-α-D-glucofuranoside

To a compound from *step a* above (13 g, 50.387 mmol), was added hydroxylamine hydrochloride (10.50 g, 151.16 mmol) pyridine (100 ml) and anhydrous ethanol (100 ml) at room temperature. The reaction mixture was stirred for half an hour. The temperature of the reaction was raised to 75 °C and the reaction mixture was stirred for 8-10 hours. Evaporated the solvent under reduced pressure and added toluene to remove pyridine azeotropically. Poured the contents of the reaction into ice cold water and stirred. Extracted the contents with ethyl acetate, washed with water dried over anhydrous sodium sulphate, filtered and evaporated the solvent under reduced pressure to afford the title compound as light yellow semi solid. Yield : 3.5 g.

### Step c: 1,2;5,6-Di-O-isopropylidene-3-deoxy-3-amino-α-D-allofuranoside

To a suspension of lithium aluminum hydride (0.973 g, 25.64 mmol) in freshly distilled tetrahydrofuran (50 ml) at 0 °C, was added the compound obtained from *step b* above (3.5 g, 12.82 mmol) in 50 ml tetrahydrofuran) with constant stirring. After complete addition, the reaction mixture was allowed to attain room temperature and stirred for 7-8 hours. The excess of lithium aluminum hydride was decomposed by the addition of ethyl acetate (100 ml) followed by the addition of water and sodium hydroxide solution (2 ml, 15 %) dropwise at 0 °C. The reaction mixture was filtered through pre-washed celite pad, washed with warm ethyl acetate and methanol. Extracted the reaction mixture with ethyl acetate, washed with water dried over anhydrous sodium sulphate, filtered and evaporated the solvent under reduced pressure to afford the title compound as light yellow semi solid. Yield : 2.0 g.

### Synthesis of 1,2;5,6-Di-O-isopropylidene-3-deoxy-3-amino-α-D-allofuranoside

### Step a: 1,2;5,6-Di-O-isopropylidene-3-oxo-α-D-glucofuranoside

To diacetoneglucose (25 g) (commercially available) was added dimethyl sulphoxide (100 ml) and acetic anhydride (50 ml). The reaction mixture was stirred at 50-60 °C for 24 hours. Dimethyl sulphoxide was evaporated under reduced pressure and water (2.5 ml) was added with vigorous stirring followed by the addition of ether (10 ml) and hexane. The mixture was kept in refrigerator for overnight. The solid thus separated was filtered to obtain the title compound Yield: 16 g.

### Step b: 1,2;5,6-Di-O-isopropylidene-3-deoxy-3-hydroxyimino-α-D-glucofuranoside

To a compound from *step a* above (12 g), was added hydroxylamine hydrochloride (2.5 g) pyridine (100 ml) and anhydrous ethanol (100 ml) at room temperature. The reaction mixture was stirred for half an hour. The temperature of the reaction was raised to 75 °C and the reaction mixture was stirred for 24 hours. The solvents were evaporated under reduced pressure and the residue thus obtained was poured into ice cold water. The organic product was extracted with ethyl acetate followed by washing with water, brine and dried over anhydrous sodium sulphate. The solvent was evaporated under reduced pressure and the product was purified by column chromatography using 25% ethyl acetate in hexane as eluent to furnish the title compound Yield: 8.5 g.

### Step c: 1,2;5,6-Di-O-isopropylidene-3-deoxy-3-amino-α-D-allofuranoside

To a suspension of lithium aluminum hydride (8.4 g) in tetrahydrofuran (50 ml) at 0 °C, was added the compound obtained from *step b* above (8.5 g in 50 ml tetrahydrofuran) with constant stirring. After complete addition, the reaction mixture was allowed to attain room temperature and stirred for 8 hours. The excess of lithium aluminum hydride was decomposed by the addition of ethyl acetate (100 ml) followed by the addition of water and sodium hydroxide solution (2 ml, 15 %) dropwise at 0 °C. The reaction mixture was filtered off, washed with warm ethyl acetate and dried over anhydrous sodium sulphate. The solvent was evaporated under reduced pressure and the crude product thus obtained was purified by column chromatography using 50% methanol in ethyl acetate as eluent to furnish the title compound Yield: 7.0 g.

### Scheme I:

### Example 1: Synthesis of 1,2;5,6-Di-O-isopropylidene-3-deoxy-3-(phenyl-propionamido)-α-D-glucofuranoside (Compound No. 31)

To a solution of the compound 1,2;5,6-Di-O-isopropylidene-3-deoxy-3-amino-α-D-glucofuranoside (0.300 g, 1.158 mmol) in dry dimethylformamide (5 ml) was added *N-*methylmorpholine (0.351 g, 3.474 mmol) and 3-phenylpropionic acid (0.208 g, 1.389 mmol). The mixture was stirred at room temperature for 10 minutes. To the resulting reaction mixture was added 1-hydroxybenzotriazole (0.313 g, 2.316 mmol) and stirred at room temperature for 1 hour. To it was added 1-(3-dimethylaminopropyl)-3-ethylcarbodiimide hydrochloride (0.444 g, 2.316 mmol) at 0 °C and stirred at the same temperature for 1 hour and subsequently at room temperature for 12 hours. The reaction mixture was poured into water, extracted with ethyl acetate, washed with water, dried over anhydrous sodium sulphate, filtered and concentrated under reduced pressure. The residue thus obtained was purified by preparative thin layer chromatography using 50% ethyl acetate in hexane as eluent to furnish the title compound. Yield: 0.45 g.
¹H NMR (CDCl₃ + D₂O exchange, 400 MHz): δ 7.32-7.19 (m, 5H, Ar-H), 6.06 (brs, 1H, -NH & D₂O exchangeable), 5.60-5.59 (d, 1H, J = 4.00 Hz, -CH), 4.35-4.33 (m, 2H, 2x-CH), 4.23-4.22 (m, 1H, -CH), 4.13-4.11 (m, 1H, -CH), 4.08-4.04 (m, 1H, -CH), 3.83-3.78 (m, 1H, -CH), 2.99-2.95 (t, 2H, J= 8.00 Hz, -CH₂Ar), 2.54-2.43 (m, 2H, -CH₂CO), 1.49 (s, 3H, -CH₃), 1.41 (s, 3H, -CH₃), 1.33 (s, 3H,- CH₃) and 1.27 (s, 3H,- CH₃)
Mass (m/z, +ve ion mode): 392 [M⁺+1].
Following compounds were prepared analogously,
1,2;5,6-O-isopropylidene-3-deoxy-3-[(2-methoxyphenyl)-propionamido]-α-D-glucofuranoside (Compound No. 32);
Mass (m/z, +ve ion mode): 422 [M⁺+1];
1,2;5,6-Di-O-isopropylidene-3-deoxy-3-[(4-fluorophenyl)-propionamido]-α-D-glucofuranoside (Compound No. 33);
Mass (m/z, +ve ion mode): 410 [M⁺+1];
1,2;5,6-Di-O-isopropylidene-3-deoxy-3-[(2-phenyl)propionamido]-α-D-glucofuranoside (Compound No. 103);
Mass (m/z, +ve ion mode): 393 [M⁺+1];
1,2;5,6-Di-O-isopropylidene-3-deoxy-3-[3-(1,3-benzodioxol-5-yl) propionamido]-α-D-glucofuranoside (Compound No. 104);
Mass (m/z, +ve ion mode): 436 [M⁺+1];
1,2;5,6-Di-O-isopropylidene-3-deoxy-3-[(3-bromobenzyl)-amido]-α-D-glucofuranoside (Compound No. 108);
Mass (m/z, +ve ion mode): 458 [M⁺+1+2] and 456 [M⁺+1]
1,2;5,6-Di-O-isopropylidene-3-deoxy-3-[(3-fluorobenzyl)-amido]-α-D-allofuranoside (Compound No. 111);
Mass (m/z, +ve ion mode): 396 [M⁺+1].

### Example 2: Synthesis of 1,2;5,6-Di-O-isopropylidene-3-deoxy-3-[(2-trifluoromethylphenyl)-urido]-α-D-glucofuranoside (Compound No. 41)

To a solution of the compound 1,2;5,6-Di-O-isopropylidene-3-deoxy-3-amino-α-D-glucofuranoside (0.500 g, 1.1930 mmol) in dichloromethane (5 ml) was added triethyl amine (0.645 ml, 4.633 mmol) and 2-trifluoromethylphenyl isocyanate (0.433 g, 2.316 mmol) and stirred the reaction mixture at room temperature for 6-7 hours. The solvent was evaporated under reduced pressure and the residue thus obtained was treated with dichloromethane and water. The organic layer was separated, washed with water and brine, dried over anhydrous sodium sulphate, filtered and concentrated under reduced pressure. The residue thus obtained was purified by column chromatography using 20% ethyl acetate in hexane as eluent to furnish the title compound. Yield: 0.838 g.
¹H NMR (CDCl₃+MeOD, 400 MHz): δ 7.95-7.93 (d 1H, J= 8.00 Hz, Ar-H), 7.53-7.51 (m, 2H, Ar-H), 7.59-7.51 (m, 2H, Ar-H), 7.20-7.09 (m, 1H, Ar-H), 5.87-5.86 (d, 1H, J = 4.00 Hz, -CH), 4.60-4.59 (m, 1H, J = 4.00 Hz, -CH), 4.43-4.41 (m, 1H, -CH), 4.26-4.23 (m, 1H, -CH), 4.12-4.08 (m, 1H, -CH), 4.01-3.98 (m, 1H, -CH), 1.59 (s, 3H, -CH₃), 1.50 (s, 3H, -CH₃) and 1.34-1.33 (m, 6H, 2x-CH₃).
Mass (m/z, +ve ion mode): 446 [M⁺+1].
Following compounds were prepared analogously by using appropriate cyanate or isocyanate, as applicable in each case,
1,2;5,6-Di-O-isopropylidene-3-deoxy-3-[(2-methoxyphenyl)-urido]-α-D-glucofuranoside (Compound No. 42);
Mass (m/z, +ve ion mode): 409 [M⁺+1];
1,2;5,6-Di-O-isopropylidene-3-deoxy-3-[(3-fluorophenyl)-thiourido]-α-D-glucofuranoside (Compound No. 43);
Mass (m/z, +ve ion mode): 413 [M⁺+1];
1,2;5,6-Di-O-isopropylidene-3-deoxy-3-[(3-trifluoromethylphenyl)-thiourido]-α-D-glucofuranoside (Compound No. 44);
Mass (m/z, +ve ion mode): 463 [M⁺+1];
1,2;5,6-Di-O-isopropylidene-3-deoxy-3-(phenyl-urido)-α-D-glucofuranoside (Compound No. 107);
1,2;5,6-Di-O-isopropylidene-3-deoxy-3-[(3-fluorophenyl)-urido]-α-D-allofuranoside (Compound No. 85);
Mass (m/z, +ve ion mode): 397 [M⁺+1];
1,2;5,6-Di-O-isopropylidene-3-deoxy-3-[(3-fluorophenyl)-thiourido]-α-D-allofuranoside (Compound No. 88);
Mass (m/z, +ve ion mode): 413 [M⁺+1];
1,2;5,6-Di-O-isopropylidene-3-deoxy-3-[(4-fluorophenyl)-urido]-α-D-allofuranoside (Compound No. 110);
1,2;5,6-Di-O-isopropylidene-3-deoxy-3-[(3-cyanophenyl)-thiourido]-α-D-glucofuranoside (Compound No. 130);
1,2;5,6-Di-O-isopropylidene-3-deoxy-3-[(2-nitrophenyl)-thiourido]-α-D-glucofuranoside (Compound No. 131);
Mass (m/z, +ve ion mode): 462 [M⁺+23];
1,2;5,6-Di-O-isopropylidene-3-deoxy-3-[(2,6-dimethylphenyl)-thiourido]-α-D-glucofuranoside (Compound No. 132);
Mass (m/z, +ve ion mode): 423 [M⁺+1];
1,2;5,6-Di-O-isopropylidene-3-deoxy-3-[(2-methoxyphenyl)-thiourido]-α-D-glucofuranoside (Compound No. 133).
Mass (m/z, +ve ion mode): 425 [M⁺+1];

### Example 3: Synthesis of 1,2;5,6-Di-O-isopropylidene-3-deoxy-3-[3-(2-nitrophenyl)-4-methyl-2-thioxo-2,3-dihydro-1H-imidazol-1-yl]-α-D-glucofuranoside (Compound No. 121)

To a solution of the compound No.19 (0.300 g, 0.683 mmol) in dry acetone (5 ml) was added triethylamine (0.09 ml, 0.700 mmol) and a solution of bromine (0.036 ml) in acetone (10 ml) drop wise under a nitrogen atmosphere and stirred at room temperature till the completion of reaction. The reaction mixture was filtered through celite pad and concentrated under reduced pressure. The residue thus obtained was purified by preparative TLC using 50% ethyl acetate and hexane as eluant to furnish the title compound. Yield: 0.200 g.
¹H NMR (CDCl₃, 400 MHz): δ 7.85-7.88 (m, 1H), 7.45-7.46 (m, 1H), 7.00-7.12 (m, 1H), 6.98-7.00 (m, 1H), 5.94-5.95 (d, 1H, J = 4.0 Hz), 5.33-5.34 (d, 2H, J = 4.0 Hz), 5.08 (s, 1H), 4.21-4.23 (m, 3H), 4.12-4.13 (m, 2H), 3.17-3.23 (m, 1H), 1.63 (s, 3H), 1.27-1.54 (m, 12H).
Mass (m/z, +ve ion mode): 478 [M⁺+1].

Following compounds were prepared analogously by reacting appropriate thiourido compound with the compound of Formula VIIb, respectively, as applicable in each case:
1,2;5,6-Di-O-isopropylidene-3-deoxy-3-[3-(3-cyanophenyl)-4-methyl-2-thioxo-2,3-dihydro-1*H*-imidazol-1-yl]-α-D-glucofuranoside (Compound No. 120);
Mass (m/z, +ve ion mode): 404 [M⁺+1];
1,2;5,6-Di-O-isopropylidene-3-deoxy-3-[3-(4-fluorophenyl)-4-methyl-2-thioxo-2,3-dihydro-1*H*-imidazol-1-yl]-α-D-glucofuranoside (Compound No. 122);
Mass (m/z, +ve ion mode): 451 [M⁺+1];
1,2;5,6-Di-O-isopropylidene-3-deoxy-3-[3-(2,6-dimethylphenyl)-4-methyl-2-thioxo-2,3-dihydro-1*H*-imidazol-1-yl]-α-D-glucofuranoside (Compound No. 123);
Mass (m/z, +ve ion mode): 461[M⁺+1];
1,2;5,6-Di-O-isopropylidene-3-deoxy-3-(3-phenyl-4-methyl-2-thioxo-2,3-dihydro-1*H-*imidazol-1-yl]-α-D-glucofuranoside (Compound No. 124);
Mass (m/z, +ve ion mode): 433 [M⁺+1];
1,2;5,6-Di-O-isopropylidene-3-deoxy-3-[3-(2-methoxyphenyl)-4-methyl-2-thioxo-2,3-dihydro-1*H*-imidazol-1-yl]-α-D-glucofuranoside (Compound No. 125);
Mass (m/z, +ve ion mode): 463[M⁺+1];
1,2;5,6-Di-O-isopropylidene-3-deoxy-3-(3-phenyl-2-thioxo-2,3-dihydro-1*H*-imidazo1-1-yl]-α-D-glucofuranoside (Compound No. 126);
Mass (m/z, +ve ion mode): 419 [M⁺+1].

### Example 4: Synthesis of 1,2-O-isopropylidene-3-deoxy-3-(phenyl-urido)-α-D-glucofuranoside (Compound No. 37)

To a solution of 1,2;5,6-Di-O-isopropylidene-3-deoxy-3-(phenyl-urido)-α-D-glucofuranoside (Compound No. 107) (0.712 g, 1.883 mmol) in tetrahydrofuran (5 ml) was added aqueous perchloric acid (60%, 0.5 M, 3.76 ml, 1.883 mmol) dropwise at 0 °C and stirred the reaction mixture at this temperature for 2 hours. The reaction mixture was quenched with a saturated solution of sodium bicarbonate and extracted with ethyl acetate. The organic layer was separated, washed with water and brine, dried over anhydrous sodium sulphate and filtered. The filtrate was evaporated under reduced pressure and the crude product thus obtained was purified by preparative TLC using 70% ethyl acetate in hexane as eluent to furnish the title compound. Yield:.0.461 g.
¹H NMR (MeOD, 400 MHz): δ 7.76-7.75 (m, 2H, Ar-H), 7.36-7.34 (m, 2H, Ar-H), 6.99-6.97 (m, 1H, Ar-H), 5.87-5.86 (d, 1H, J = 4.00 Hz, -CH), 4.58-4.57 (d, 1H, J = 4.00 Hz, -CH), 4.31-4.30 (d, 1H, 1H, J = 4.00 Hz, -CH), 4.16-4.13 (m, 1H, -CH), 3.78-3.72 (m, 2H, 2x-CH), 3.61-3.57 (m, 1H, -CH), 1.48 (s, 3H, -CH₃) and 1.31 (s, 3H, -CH₃)
Mass (m/z, +ve ion mode): 339 [M⁺+1].
Following compounds were prepared analogously,
1,2-O-isopropylidene-3-deoxy-3-[(4-fluorophenyl)-urido]-α-D-glucofuranoside (Compound No. 38);
Mass (m/z, +ve ion mode): 379 [M⁺+23];
1,2-O-isopropylidene-3-deoxy-3-[(2-fluorophenyl)-urido]-α-D-glucofuranoside (Compound No. 39);
Mass (m/z, +ve ion mode): 357 [M⁺+1];
1,2-O-isopropylidene-3-deoxy-3-[(4-cyanophenyl)-urido]-α-D-glucofuranoside (Compound No. 40);
Mass (m/z, +ve ion mode): 364 [M⁺+1];
1,2-O-isopropylidene-3-deoxy-3-[(4-cyanophenyl)-thiourido]-α-D-glucofuranoside (Compound No. 45);
1,2-O-isopropylidene-3-deoxy-3-[3-fluorophenyl)-thiourido]-α-D-glucofuranoside (Compound No. 46);
Mass (m/z, +ve ion mode): 373 [M⁺+1];
1,2-O-isopropylidene-3-deoxy-3-[(3-trifluoromethylphenyl)-thiourido]-α-D-glucofuranoside (Compound No. 47);
Mass (m/z, +ve ion mode): 423 [M⁺+1];
1,2-O-isopropylidene-3-deoxy-3-[(3-fluorophenyl)-thiourido]-α-D-allofuranoside (Compound No. 93);
Mass (m/z, +ve ion mode): 373 [M⁺+1];
1,2-O-isopropylidene-3-deoxy-3-[(3-bromobenzyl)-amido]-α-D-glucofuranoside (Compound No. 109);
Mass (m/z, +ve ion mode): 418 [M⁺+1+2] and 416 [M⁺+1];
1,2-O-isopropylidene-3-deoxy-3-[(3-fluorobenzyl)-amido]-α-D-allofuranoside (Compound No. 112;
Mass (m/z, +ve ion mode): 356 [M⁺+1].

### Example 5: Synthesis of 1,2-O-isopropylidene-3-deoxy-3-[(3-bromobenzyl)-amido]-5,6-dioxa spiro[4,4]nonane-α-D-glucofuranoside (Compound No. 1)

To a solution of 1,2-O-isopropylidene-3-deoxy-3-[(3-bromobenzyl)-amido]-α-D-glucofuranoside (Compound No. 109) (0.100 g, 0.240 mmol) in a mixture of dry toluene (5 ml) and dry dimethylformamide (2 ml) was added dry and powdered molecular sieves (0.250 g) and cyclopentanone dimethyl ketal (0.35 g, 2.403 mmol) and heated the contents at 120-130 °C for 8-10 hours. Filtered the reaction mixture through a celite pad, prewashed with methanol and washed the celite with ethyl acetate several times. Evaporated the solvents under reduced pressure to afford a residue which was then purified through preparative TLC using 50% ethyl acetate in hexane as eluent to furnish the title compound. Yield: 0.050 g.
¹H NMR (CDCl₃ + D₂O exchange, 400 MHz): δ 7.72 -7.43 (m, 2H, Ar-H), 7.31-7.21 (m, 2H, Ar-H), 6.09-6.07 (brd, 1H, -NH & D₂O exchangeable), 5.81-5.80 (d, 1H, J = 4.00 Hz, -CH), 4.60-4.59 (d, 1H, J = 4.00 Hz, -CH), 4.38-4.36 (m, 1H, 1H, -CH), 4.16-4.13 (m, 1H, -CH), 4.03-4.00 (m, 2H, 2x-CH), 3.81-3.80 (m, 1H, -CH), 3.52 (s, 2H, -CH₂Ar), 1.84-1.81 (m, 2H, -CH₂), 1.71-1.68 (m, 6H, 3x-CH₂), 1.50 (s, 3H, -CH₃) and 1.28 (s, 3H, -CH₃)
Mass (m/z, +ve ion mode): 484 [M⁺+1+2] and 482 [M⁺+1].
Following compounds were prepared analogously,
1,2-O-isopropylidene-3-deoxy-3-[(3-chlorobenzyl)-amido]-5,6-dioxaspiro[4,4]nonane-α-D-glucofuranoside (Compound No. 2);
Mass (m/z, +ve ion mode): 440 [M⁺+1+2] and 438[M⁺+1];
1,2-O-isopropylidene-3-deoxy-3-[(2,4-difluorobenzyl)-amido]-5,6-dioxaspiro[4,4]nonane-α-D-glucofuranoside (Compound No. 3);
Mass (m/z, +ve ion mode): 440 [M⁺+1];
1,2-O-isopropylidene-3-deoxy-3-[(2-chloro-4-fluorobenzyl)-amido]-5,6-dioxa spiro[4,4]nonane-α-D-glucofuranoside (Compound No. 4);
Mass (m/z, +ve ion mode): 458 [M⁺+1+2] and 456[M⁺+1];
1,2-O-isopropylidene-3-deoxy-3-[(3-fluorobenzyl)-amido]-5,6-dioxaspiro[4,4]nonane-α-D-glucofuranoside (Compound No. 5);
Mass (m/z, +ve ion mode): 422 [M⁺+1];
1,2-O-isopropylidene-3-deoxy-3-[(3,4,5-trimethoxybenzyl)-amido]-5,6-dioxa spiro[4,4]nonane-α-D-glucofuranoside (Compound No. 6);
Mass (m/z, +ve ion mode): 494 [M⁺+1];
1,2-O-isopropylidene-3-deoxy-3-[(2,5-dimethylbenzyl)-amido]-5,6-dioxa spiro[4,4]nonane-α-D-glucofuranoside (Compound No. 7);
Mass (m/z, +ve ion mode): 432[M⁺+1];
1,2-O-isopropylidene-3-deoxy-3-[(4-fluorophenyl)-urido]-5,6-dioxa spiro[4,5]decane-α-D-glucofuranoside (Compound No. 48);
Mass (m/z, +ve ion mode): 437 [M⁺+1];
1,2-O-isopropylidene-3-deoxy-3-[(2-fluorophenyl)-urido]-5,6-dioxa spiro[4,5]decane-α-D-glucofuranoside (Compound No. 49);
Mass (m/z, +ve ion mode): 437[M⁺+1];
1,2-O-isopropylidene-3-deoxy-3-[(2-trifluoromethylphenyl)-urido]-5,6-dioxa spiro[4,5]decane-α-D-glucofuranoside (Compound No. 50);
Mass (m/z, +ve ion mode): 487 [M⁺+1];
1,2-O-isopropylidene-3-deoxy-3-[(4-cyanophenyl)-urido]-5,6-dioxa spiro[4,5]decane-α-D-glucofuranoside (Compound No. 51);
Mass (m/z, +ve ion mode): 444 [M⁺+1];
1,2-O-isopropylidene-3-deoxy-3-(octyl-urido)-5,6-dioxaspiro[4,5]decane-α-D-glucofuranoside (Compound No. 52);
Mass (m/z, +ve ion mode): 455 [M⁺+1];
1,2-O-isopropylidene-3-deoxy-3-(phenyl-urido)-5,6-dioxa spiro[4,5]decane-α-D-glucofuranoside (Compound No. 53);
Mass (m/z, +ve ion mode): 419 [M⁺+1];
1,2-O-isopropylidene-3-deoxy-3-[(2-methoxyphenyl)-urido]-5,6-dioxa spiro[4,5]decane-α-D-glucofuranoside (Compound No. 54);
Mass (m/z, +ve ion mode): 449 [M⁺+1];
1,2-O-isopropylidene-3-deoxy-3-[(4-trifluoromethylphenyl)-urido]-5,6-dioxa spiro[4,5]decane-α-D-glucofuranoside (Compound No. 55);
Mass (m/z, +ve ion mode): 487 [M⁺+1];
1,2-O-isopropylidene-3-deoxy-3-[(3-methoxyphenyl)-thiourido]-5,6-dioxa spiro[4,5]decane-α-D-glucofuranoside (Compound No. 56);
Mass (m/z, +ve ion mode): 465 [M⁺+1];
1,2-O-isopropylidene-3-deoxy-3-[(3-fluorophenyl)-thiourido]-5,6-dioxa spiro[4,5]decane-α-D-glucofuranoside (Compound No. 57);
Mass (m/z, +ve ion mode): 453 [M⁺+1];
1,2-O-isopropylidene-3-deoxy-3-[(3-trifluoromethylphenyl)-thiourido]-5,6-dioxa spiro[4,5]decane-α-D-glucofuranoside (Compound No. 58);
Mass (m/z, +ve ion mode): 503 [M⁺+1];
1,2-O-isopropylidene-3-deoxy-3-[(3-methoxyphenyl)-urido]-5,6-dioxa spiro[4,5]decane-α-D-glucofuranoside (Compound No. 59).
Mass (m/z, +ve ion mode): 449 [M⁺+1].

### Scheme II:

### Example 6: Synthesis of 1,2;5,6-Di-O-isopropylidene-3-deoxy-3-(phenyl-propionamido)-α-D-allofuranoside (Compound No. 34)

To a solution of the compound 1,2;5,6-Di-O-isopropylidene-3-deoxy-3-amino-α-D-allofuranoside (0.300 g, 1.158 mmol) in dry dimethylformamide (5 ml) was added *N-*methylmorpholine (0.351 g, 3.474 mmol) and 3-phenylpropionic acid (0.208 g, 1.389 mmol). The mixture was stirred at room temperature for 10 minutes. To the resulting reaction mixture was added 1-hydroxybenzotriazole (0.313 g, 2.316 mmol) and stirred at room temperature for 1 hour. To it was added 1-(3-dimethylaminopropyl)-3-ethylcarbodiimide hydrochloride (0.444 g, 2.316 mmol) at 0 °C and stirred the reaction mixture at the same temperature for 1 hour and subsequently at room temperature for 12 hours. The reaction mixture was poured into water, extracted with ethyl acetate, washed with water, dried over anhydrous sodium sulphate, filtered and concentrated under reduced pressure. The residue thus obtained was purified by preparative thin layer chromatography using 40 % ethyl acetate in hexane as eluent to furnish the title compound. Yield: 0.320 g.
¹H NMR (CDCl₃ + D₂O exchange, 400 MHz): δ 7.32-7.20 (m, 5H, Ar-H), 5.82-5.81 (d, 1H, J = 4.00 Hz, -CH), 5.75 (brs, 1H, -NH & D₂O exchangeable), 4.56-4.54 (t, 1H, J = 4.00 Hz, -CH), 4.18-4.15 (m, 2H, 2x-CH), 4.09-4.05 (m, 1H, -CH), 3.92-3.90 (m, 1H, CH), 3.83-3.80 (m, 1H, -CH), 2.97-2.95 (t, 2H, J= 8.00 Hz, -CH₂Ar), 2.56-2.53 (m, 2H, -CH₂CO), 1.52 (s, 3H, -CH₃), 1.43 (s, 3H, -CH₃), 1.34 (s, 3H, -CH₃) and 1.32 (s, 3H, -CH₃)
Mass (m/z, +ve ion mode): 414 [M⁺+23].
Following compounds were prepared analogously,
1,2;5,6-Di-O-isopropylidene-3-deoxy-3-[(2-methoxyphenyl)-propionamido]-α-D-allofuranoside Compound No. 35);
Mass (m/z, +ve ion mode): 444 [M⁺+23];
1,2;5,6-Di-O-isopropylidene-3-deoxy-3-[(4-fluorophenyl)-propionamido]-α-D-allofuranoside (Compound No. 36);
1,2;5,6-Di-O-isopropylidene-3-deoxy-3-[(2-phenyl)-propionamido]-α-D-allofuranoside (Compound No. 105);
Mass (m/z, +ve ion mode): 414 [M⁺+23];
1,2;5,6-Di-O-isopropylidene-3-deoxy-3-[3-(1,3-benzodioxol-5-yl)-propionamido]-α-D-allofuranoside (Compound No. 106);
Mass (m/z, +ve ion mode): 458 [M⁺+23].

### Example 7: Synthesis of 1,2;5,6-Di-O-isopropylidene-3-deoxy-3-[(4-trifluoromethyphenyl)-urido]-α-D-allofuranoside (Compound No. 62)

The title compound was prepared following the procedure as described in Example 2, by using the compound 1,2;5,6-Di-O-isopropylidene-3-deoxy-3-amino-α-D-allofuranoside in place of 1,2;5,6-Di-O-isopropylidene-3-deoxy-3-amino-α-D-glucofuranoside.
¹H NMR (CDCl₃ + D₂O exchange, 400 MHz): δ 7.61-7.49 (m, 4H, Ar-H), 6.28-6.26 (m, 1H, -NH & D₂O exchangeable), 5.87-5.86 (d, 1H, J = 4.00 Hz, -CH), 4.68-4.66 (m, 1H, -CH), 4.33-4.32 (m, 1H, -CH), 4.15-4.11 (m, 2H, 2x-CH), 4.02-3.93 (m, 2H, 2x-CH), 1.58 (s, 3H, -CH₃), 1.44 (s, 3H, -CH₃), 1.36 (s, 3H,- CH₃) and 1.35 (s, 3H,- CH₃)
Mass (m/z, +ve ion mode): 447 [M⁺+1].
Following compounds were prepared similarily by coupling an amine with appropriate cyanate or isocyanate, as applicable in each case.
1,2;5,6-Di-O-isopropylidene-3-deoxy-3-(phenyl-urido)-α-D-allofuranoside (Compound No. 63);
Mass (m/z, +ve ion mode): 379 [M⁺+1];
1,2;5,6-Di-O-isopropylidene-3-deoxy-3-[(2-methoxyphenyl)-urido]-α-D-allofuranoside (Compound No. 64);
Mass (m/z, +ve ion mode): 409 [M⁺+1];
1,2;5,6-Di-O-isopropylidene-3-deoxy-3-[4-cyanophenyl)-urido]-α-D-allofuranoside (Compound No. 65);
Mass (m/z, +ve ion mode): 404 [M⁺+1];
1,2;5,6-Di-O-isopropylidene-3-deoxy-3-[3-trifluoromethylphenyl)-thiourido]-α-D-allofuranoside (Compound No. 89);
Mass (m/z, +ve ion mode): 463 [M⁺+1].

### Example 8: Synthesis of 1,2-O-isopropylidene-3-deoxy-3-[4-fluorophenyl)-urido]-α-D-allofuranoside (Compound No. 60)

To a solution of 1,2;5,6-Di-O-isopropylidene-3-deoxy-3-[(4-fluorophenyl)-urido]-α-D-allofuranoside (Compound No. 110) (0.617 g, 1.558 mmol) in tetrahydrofuran (7 ml) was added aqueous perchloric acid (60%, 0.5 M, 0.260 ml, 1.558 mmol) dropwise at 0 °C and stirred the reaction mixture at this temperature for 2 hours. The reaction mixture was quenched with a saturated solution of sodium bicarbonate and extracted with ethyl acetate. The organic layer was washed with water, brine and filtered. The filtrate was evaporated under reduced pressure and the crude product thus obtained was purified by preparative TLC using 100% ethyl acetate as eluent to furnish the title compound. Yield: 0.325 g.
¹H NMR (CDCl₃ + D₂O exchange, 400 MHz): δ 7.31-7.20 (m, 5H, Ar-H), 5.82-5.81 (d, 1H, J = 4.00 Hz, -CH), 5.75-5.73 (brs, 1H, -NH & D₂O exchangeable), 4.56-4.53 (m, 1H, -CH), 4.18-4.15 (m, 2H, 2x-CH), 4.09-4.05 (m, 1H, -CH), 3.92-3.90 (m, 1H, -CH), 3.82-3.80 (m, 1H, -CH), 2.99-2.95 (t, 2H, J = 8.00 Hz, -CH₂Ar), 2.56-2.52 (m, 2H, -CH₂CO), 1.51 (s, 3H, -CH₃), 1.43 (s, 3H, -CH₃), 1.34 (s, 3H, -CH₃) and 1.26 (s, 3H, -CH₃).
Following compounds were prepared analogously,
1, 2-O-isopropylidene-3-deoxy-3-[(2-fluorophenyl)-urido]-α-D-allofuranoside (Compound No. 61);
Mass (m/z, +ve ion mode): 357 [M⁺+1];
1,2-O-isopropylidene-3-deoxy-3-[(4-trifluoromethylphenyl)-urido]-α-D-allofuranoside (Compound No. 66);
Mass (m/z, +ve ion mode): 407 [M⁺+1];
1,2-O-isopropylidene-3-deoxy-3-(phenyl-urido)-α-D-allofuranoside (Compound No. 67);
Mass (m/z, +ve ion mode): 339 [M⁺+1];
1,2-O-isopropylidene-3-deoxy-3-[(2-methoxyphenyl)-urido]-α-D-allofuranoside (Compound No. 68);
Mass (m/z, +ve ion mode): 369 [M⁺+1];
1,2-O-isopropylidene-3-deoxy-3-[(4-cyanophenyl)-urido]-α-D-allofuranoside (Compound No. 69);
Mass (m/z, +ve ion mode): 364 [M⁺+1];
1,2-O-isopropylidene-3-deoxy-3-[(2-trifluoromethylphenyl)-urido]-α-D-allofuranoside (Compound No. 70);
Mass (m/z, +ve ion mode): 407 [M⁺+1];
1,2-O-isopropylidene-3-deoxy-3-[(4-fluorophenyl)-thiourido]-α-D-allofuranoside (Compound No. 71);
Mass (m/z, +ve ion mode): 373 [M⁺+1];
1,2-O-isopropylidene-3-deoxy-3-[(2-fluorophenyl)-thiourido]-α-D-allofuranoside (Compound No. 72);
Mass (m/z, +ve ion mode): 373 [M⁺+1];
1,2-O-isopropylidene-3-deoxy-3-[(2-trifluoromethylphenyl)-thiourido]-α-D-allofuranoside (Compound No. 73);
Mass (m/z, +ve ion mode): 423 [M⁺+1];
1,2-O-isopropylidene-3-deoxy-3-[(2-methoxyphenyl)-thiourido]-α-D-allofuranoside (Compound No. 74);
Mass (m/z, +ve ion mode): 385 [M⁺+1];
1,2-O-isopropylidene-3-deoxy-3-(phenyl-thiourido)-α-D-allofuranoside (Compound No. 75);
Mass (m/z, +ve ion mode): 377 [M⁺+23];
1,2-O-isopropylidene-3-deoxy-3-[(4-trifluoromethylphenyl)-thiourido]-α-D-allofuranoside (Compound No. 76);
Mass (m/z, +ve ion mode): 422 [M⁺+1];
1,2-O-isopropylidene-3-deoxy-3-[(4-cyanophenyl)-thiourido]-α-D-allofuranoside (Compound No. 77);
Mass (m/z, +ve ion mode): 380 [M⁺+1];
1,2-O-isopropylidene-3-deoxy-3-[(3-fluorophenyl)-urido]-α-D-allofuranoside (Compound No. 86);
Mass (m/z, +ve ion mode): 357 [M⁺+1];
1,2-O-isopropylidene-3-deoxy-3-[(3-cyanophenyl)-urido]-α-D-allofuranoside (Compound No. 87);
Mass (m/z, +ve ion mode): 364 [M⁺+1];
1,2-O-isopropylidene-3-deoxy-3-[(3-trifluoromethylphenyl)-thiourido]-α-D-allofuranoside (Compound No. 94);
Mass (m/z, +ve ion mode): 422 [M⁺+1];
1,2-O-isopropylidene-3-deoxy-3-[(3-methoxyphenyl)-thiourido]-α-D-allofuranoside (Compound No. 95);
Mass (m/z, +ve ion mode): 385 [M⁺+1];
1,2-O-isopropylidene-3-deoxy-3-[(3-methoxyphenyl)-urido]-α-D-allofuranoside (Compound No. 102);
Mass (m/z, +ve ion mode): 369 [M⁺+1].

### Example 9: Synthesis of 1,2-O-isopropylidene-3-deoxy-3-[(3-fluorobenzyl)-amido[-5,6-dioxa spiro[4,4]nonane-α-D-allofuranoside (Compound No. 8)

To a solution of 1,2-O-isopropylidene-3-deoxy-3-[(3-fluorobenzyl)-amido]-α-D-allofuranoside (Compound No. 112) (0.100 g, 0.2801 mmol) in a mixture of dry toluene (3 ml) was added dry and powdered molecular sieves (0.250 g) and cyclopentanone dimethyl ketal (0.365 g, 2.814 mmol) and heated the contents at 110-120 °C for 8-10 hours. Filtered the reaction mixture through a celite pad prewashed with methanol and washed the celite with ethyl acetate several times. Evaporated the solvents under reduced pressure to afford a residue which was then purified through preparative TLC using 50% ethyl acetate in hexane as eluent to furnish the title compound. Yield: 0.050 g.
¹H NMR (CDCl₃, 400 MHz): δ 7.33 -7.26 (m, 2H, Ar-H), 7.07-6.98 (m, 2H, Ar-H), 5.86-5.80 (brs, 2H, -CH & -NH), 4.65-4.63 (brs, 1H, -CH), 4.16-4.09 (m, 2H, 2x-CH), 4.01-3.99 (m, 1H, -CH), 3.89-3.87 (m, 1H, -CH), 3.76-3.74 (m, 1H, -CH), 3.59 (s, 1H, -CH₂Ar), 1.78-1.67 (m, 8H, 4x-CH₂), 1.46 (s, 3H, -CH₃) and 1.31 (s, 3H, -CH₃)
Mass (m/z, +ve ion mode): 422 [M⁺+1].
Following compounds were prepared analogously,
1,2-O-isopropylidene-3-deoxy-3-[(4- fluorobenzyl)-amido]-5,6-dioxa spiro[4,4]nonane-α-D-allofuranoside (Compound No. 9);
Mass (m/z, +ve ion mode): 422 [M⁺+1];
1,2-O-isopropylidene-3-deoxy-3-[(2-chloro-4-fluorobenzyl)-amido]-5,6-dioxa spiro[4,4]nonane-α-D-allofuranoside (Compound No. 10);
Mass (m/z, +ve ion mode): 458 [M⁺+1+2] and 456 [M⁺+1];
1,2-O-isopropylidene-3-deoxy-3-[(3-bromobenzyl)-amido]-5,6-dioxa spiro[4,4]nonane-α-D-allofuranoside (Compound No. 11);
Mass (m/z, +ve ion mode): 484 [M⁺+1+2] and 482 [M⁺+1];
1,2-O-isopropylidene-3-deoxy-3-[(3-chlorobenzyl)-amido]-5,6-dioxa spiro[4,4]nonane-α-D-allofuranoside (Compound No. 12);
Mass (m/z, +ve ion mode): 440 [M⁺+1+2] and 438 [M⁺+1];
1,2-O-isopropylidene-3-deoxy-3-[(2,5-dimethylbenzyl)-amido]-5,6-dioxa spiro[4,4]nonane-α-D-allofuranoside (Compound No. 13);
Mass (m/z, +ve ion mode): 432 [M⁺+1];
1,2-O-isopropylidene-3-deoxy-3-[(3,4,5-trimethoxybenzyl)-amido]-5,6-dioxa spiro[4,4]nonane-α-D-allofuranoside (Compound No. 14);
Mass (m/z, +ve ion mode): 494 [M⁺+1];
1,2-O-isopropylidene-3-deoxy-3-[(4-fluorophenyl)-thiourido]-5,6-dioxa spiro[4,5]decane-α-D-allofuranoside (Compound No. 78);
Mass (m/z, +ve ion mode): 475 [M⁺+23];
1,2-O-isopropylidene-3-deoxy-3-[(2-fluorophenyl)-thiourido]-5,6-dioxa spiro[4,5]decane-α-D-allofuranoside (Compound No. 79);
Mass (m/z, +ve ion mode): 453 [M⁺+1];
1,2-O-isopropylidene-3-deoxy-3-[(2-trifluoromethylphenyl)-thiourido]-5,6-dioxa spiro [4,5]decane-α-D-allofuranoside (Compound No. 80);
Mass (m/z, +ve ion mode): 503 [M⁺+1];
1,2-O-isopropylidene-3-deoxy-3-(phenyl-thiourido)-5,6-dioxa spiro[4,5]decane-α-D-allofuranoside (Compound No. 81);
Mass (m/z, +ve ion mode): 435 [M⁺+1];
1,2-O-isopropylidene-3-deoxy-3-[(4-trifluoromethylphenyl)-thiourido]-5,6-dioxa spiro[4,5]decane-α-D-allofuranoside (Compound No. 82);
Mass (m/z, +ve ion mode): 503 [M⁺+1];
1,2-O-isopropylidene-3-deoxy-3-[(4-cyanophenyl)-thiourido]-5,6-dioxa spiro[4,5]decane-α-D-allofuranoside (Compound No. 83);
Mass (m/z, +ve ion mode): 460 [M⁺+1];
1,2-O-isopropylidene-3-deoxy-3-[(2-methoxyphenyl)-urido]-5,6-dioxa spiro[4,5]decane-α-D-allofuranoside (Compound No. 84);
Mass (m/z, +ve ion mode): 449 [M⁺+1];
1,2-O-isopropylidene-3-deoxy-3-[(4-trifluoromethylphenyl)-urido]-5,6-dioxa spiro[4,5]decane-α-D-allofuranoside (Compound No. 90);
Mass (m/z, +ve ion mode): 487 [M⁺+1];
1,2-O-isopropylidene-3-deoxy-3-[(2-trifluoromethylphenyl)-urido]-5,6-dioxa spiro[4,5]decane-α-D allofuranoside (Compound No. 91);
Mass (m/z, +ve ion mode): 487 [M⁺+1];
1,2-O-isopropylidene-3-deoxy-3-[(4-cyanophenyl)-urido]-5,6-dioxa spiro[4,5]decane-α-D-allofuranoside (Compound No. 92);
Mass (m/z, +ve ion mode): 444 [M⁺+1];
1,2-O-isopropylidene-3-deoxy-3-[(4-fluorophenyl)-urido]-5,6-dioxa spiro[4,5]decane-α-D-allofuranoside (Compound No. 96);
Mass (m/z, +ve ion mode): 437 [M⁺+1];
1,2-O-isopropylidene-3-deoxy-3-[(2-fluorophenyl)-urido]-5,6-dioxa spiro[4,5]decane-α-D-allofuranoside (Compound No. 97);
Mass (m/z, +ve ion mode): 437 [M⁺+1];
1,2-O-isopropylidene-3-deoxy-3-[(3-fluorophenyl)-urido]-5,6-dioxa spiro[4,5]decane-α-D-allofuranoside (Compound No. 98);
Mass (m/z, +ve ion mode): 437 [M⁺+1];
1,2-O-isopropylidene-3-deoxy-3-[(3-cyanophenyl)-urido]-5,6-dioxa spiro[4,5]decane-α-D-allofuranoside (Compound No. 99);
Mass (m/z, +ve ion mode): 444 [M⁺+1];
1,2-O-isopropylidene-3-deoxy-3-(phenyl-urido)-5,6-dioxa spiro[4,5]decane-α-D-allofuranoside (Compound No. 100);
Mass (m/z, +ve ion mode): 419 [M⁺+1];
1,2;5,6-Di-O-isopropylidene-3-deoxy-3-[(3-methoxyphenyl)-urido]-α-D-allofuranoside (Compound No. 101);
Mass (m/z, +ve ion mode): 409 [M⁺+1].

### Example 10: Synthesis of -1,2;5,6-Di-O-isopropylidene-3-deoxy-3-[3-(4-fluorohenyl)-4 methyl-2-thioxo-2,3-dihydro-1H-imidazol-1-yl]-α-D-allofuranoside (Compound No. 134)

To a solution of the compound 1,2;5,6-Di-O-isopropylidene-3-deoxy-3-{[(4-fluorophenyl)-amino]thiocarbonyl}-amino-α-D-allofuranoside (disclosed in our co-pending Indian patent application No. 875/DEL/2006) (0.150 g, 0.364 mmol) in dry acetone (5 ml) was added triethylamine (0.05 ml, 0.364 mmol) and a solution of bromine (0.018 ml) in acetone (10 ml) dropwise under a nitrogen atmosphere and stirred the reaction mixture at room temperature till the completion of reaction. The reaction mixture was filtered through celite pad and concentrated under reduced pressure. The residue thus obtained was purified by preparative TLC using 50% ethyl acetate and hexane as eluant to furnish the title compound. Yield: 0.110 g.
¹H NMR (CDCl₃, 400 MHz): δ 7.22-7.26 (m, 2H), 7.10-7.14 (m, 2H), 5.84-5.85 (d, 1H, J = 4.0 Hz), 5.64-5.63 (d, 1H, J = 1.2 Hz), 4.92-4.90 (m, 1H), 4.39-4.43 (m, 1H), 4.15-4.18 (m, 1H), 3.81-3.83 (m, 2H), 3.44-3.47 (m, 1H), 1.79 (s, 3H), 1.44 (s, 3H), 1.39 (s, 3H), 1.34 (s, 3H) and 1.30 (s, 3H).
Mass (m/z, +ve ion mode): 451 [M⁺+1].
Following compounds were prepared similarly:,
1,2;5,6-Di-O-isopropylidene-3-deoxy-3-[3-(4-cyanophenyl)-4-methyl-2-thioxo-2,3-dihydro-1*H*-imidazol-1-yl]-α-D-allofuranoside (Compound No. 113);
Mass (m/z, +ve ion mode): 458 [M⁺+1];
1,2;5,6-Di-O-isopropylidene-3-deoxy-3-[3-(2-fluorophenyl)-4-methyl-2-thioxo-2,3-dihydro-1*H*-imidazol-1-yl]-α-D-allofuranoside (Compound No. 114);
Mass (m/z, +ve ion mode): 451 [M⁺+1 ];
1,2;5,6-Di-O-isopropylidene-3-deoxy-3-[3-(2-methoxyphenyl)-4-methyl-2-thioxo-2,3-dihydro-1*H*-imidazol-1-yl]-α-D-allofuranoside (Compound No. 115);
Mass (m/z, +ve ion mode): 463 [M⁺+1];
1,2;5,6-Di-O-isopropylidene-3-deoxy-3-[3-(2,6-dimethylphenyl)-4-methyl-2-thioxo-2,3-dihydro-1*H*-imidazol-1-yl]-α-D-allofuranoside (Compound No. 116);
Mass (m/z, +ve ion mode): 461 [M⁺+1];
1,2;5,6-Di-O-isopropylidene-3-deoxy-3-[3-(3-methoxyphenyl)-4-methyl-2-thioxo-2,3-dihydro-1*H*-imidazol-1-yl]-α-D-allofuranoside (Compound No. 117);
Mass (m/z, +ve ion mode): 463 [M⁺+1];
1,2;5,6-Di-O-isopropylidene-3-deoxy-3-(3-phenyl-4-methyl-2-thioxo-2,3-dihydro-1*H-*imidazol-1-yl]-α-D-allofuranoside (Compound No. 118);
Mass (m/z, +ve ion mode): 433 [M⁺+1];
1,2;5,6-Di-O-isopropylidene-3-deoxy-3-[3-(2-trifluoromethylphenyl)-4-methyl-2-thioxo-2,3-dihydro-1*H*-imidazol-1-yl]-α-D-allofuranoside (Compound No. 119);
Mass (m/z, +ve ion mode): 501 [M⁺+1];

### Example 11: Synthesis of 1,2;5,6-Di-O-isopropylidene-3-deoxy-3-[(2-methoxybenzyl)-thioamidol-α-D-allofuranoside (Compound No. 129)

To a solution of 1,2;5,6-Di-O-isopropylidene-3-O-[(2-methoxybenzyl)-amido]-carbonyl]-α-D-allofuranoside (disclosed in our co-pending Indian patent application No. 875/DEL/2006) (0.212 g, 0.521 mmol) in dry distilled toluene (4 ml) was added Lawesson's reagent (0.126 g, 0.311 mmol) and stirred at room temperature overnight. Evaporated the solvent under vacuum to afford a yellow residue which was purified by column chromatography over basic alumina using 10% ethyl acetate and hexane as eluant to afford the desired product. Yield: 0.055 g.
¹H NMR (CDCl₃, 400 MHz): δ 8.15-8.17 (d, 1H, 8.0 Hz), 7.26-7.31 (m, 2H), 6.90-6.98 (m, 2H), 5.83-5.84 (d, 1H, J = 4.0 Hz), 4.80-4.90 (m, 1H), 4.64-4.66 (m, 1H), 4.23-4.24 (m, 1H), 4.06-4.13 (m, 4H), 3.87-3.91 (m, 5H), 1.46 (s, 3H), 1.34 (s, 3H), 1.30 (s, 3H) and1.28 (s, 3H).
Mass (m/z, +ve ion mode): 424 [M⁺+1].
Following compounds were prepared analogously:
1,2;5,6-Di-O-isopropylidene-3-deoxy-3-[(4-fluorobenzyl)-thioamido]-α-D-allofuranoside (Compound No. 127); Mass (m/z, +ve ion mode): 412 [M⁺+1];
1,2;5,6-Di-O-isopropylidene-3-deoxy-3-[(2-chloro-4-fluorobenzyl)-thioamido]-α-D-allofuranoside (Compound No. 128);
Mass (m/z, +ve ion mode): 448 [M⁺+1+2] and [M⁺+1].

### Scheme III:

### Example 12: Synthesis of 1,2-Dioxa spiro[2,5]octane-3-deoxy-3-[(4-fluorophenyl)-thiourido]-5,6-O-isopropylidene-α-D-allofuranoside (Compound No. 20)

### Step a: 1,2-Dioxa spiro[2,5]octane-5,6-O-isopropylidene-α-D-glucofuranoside

To a solution of 1,2-Dioxa spiro[2,5]octane-α-D-glucofuranoside (Commercially available) (2.5 g, 9.605 mmol) in dry toluene (20 ml) was added powdered molecular sieves (5 g), excess 2,2-dimethoxy propane (25 ml) and catalytic amount of para toluenesulphonic acid. Refluxed the contents of the reaction for 1 hour. Filtered the reaction mixture through a celite pad and washed with ethyl acetate. Evaporated the solvent under reduced pressure and the residue so obtained was triturated with hexane to obtain the above product as white fluffy solid. Yield : 2.65 g.

### Step b: 1,2-Dioxa spiro[2,5]octane-3-oxo-5,6-O-isopropylidene-α-D-glucofuranoside

To a solution of the compound obtained from step *a* above (2.60 g, 8.666 mmol) in dry distilled dimethyl sulphoxide (20 ml), added acetic anhydride (10 ml, 104.0 mmol) and heated the contents of the reaction at 70-80 °C for 5 hours. Added water to it and stirred at room temperature. Extracted the reaction mixture with ethyl acetate, washed with water, dried over anhydrous sodium sulphate, filtered and evaporated the solvent under reduced pressure to obtain the above product as thick brown oil. Yield: 4.0 g.

### Step c: 1, 2-Dioxa spiro[2,5]octane-3-deoxy-3-hydroxyimino-5,6-O-isopropylidene-α-D-glucofuranoside

To a solution of the compound obtained from step *b* above (4.0 g, 13.42 mmol) in dry ethanol (20 ml) and dry pyridine (20 ml) added hydroxylamine hydrochloride (2.79 g, 40.26 mmol) and heated the contents of the reaction at 70-80 °C for 7-8 hours. Evaporated the solvents under reduced pressure and added toluene to remove pyridine azeotropically. Extracted the reaction mixture with dichloromethane, washed with water, dried over anhydrous sodium sulphate, filtered and evaporated the solvent under reduced pressure to obtain the above product as thick brown oil. Yield: 4.00 g. This product was used as such for the next step without purification.

### Step d: 1,2-Dioxa spiro[2,5]octane-3-deoxy-3-amino-5,6-O-isopropylidene-α-D-allofuranoside

To a suspension of lithium aluminium hydride (0.728 g, 19.169 mmol) in dry distilled tetrahydrofuran (30 ml) cooled to 0 °C was added a solution of compound obtained from step c above (3.00 g) in dry distilled tetrahydrofuran (5 ml). Stirred the reaction mixture at 0 °C for 2 hours and then at room temperature for 1 hour. Cooled the reaction mixture to 0 °C and added ethyl acetate dropwise. A saturated solution of sodium sulphate was then added slowly under stirring at 0 °C till a white gelatinous precipitate was formed. Filtered the reaction mixture through a celite pad and washed with ethyl acetate. Added water to it and extracted with ethyl acetate. Washed the organic layer with water, dried over anhydrous sodium sulphate, filtered and evaporated the solvent under reduced pressure to obtain the above product as yellow oil. Yield: 2.20 g.

### Step e: 1,2-Dioxa spiro[2,5]octane-3-deoxy-3-[(4-fluorophenyl)-thiourido]-5,6-O-isopropylidene-α-D-allofuranoside

To a solution of compound obtained from step *d* above (0.100 g, 3.344 mmol) in dichloromethane (3.0 ml) was added triethyl amine (1.398 ml, 10.033 mmol) and 4-fluoromethylphenyl isocyanate (0.1024 g, 6.688 mmol) and stirred the reaction mixture at room temperature for 6-7 hours. The solvent was evaporated under reduced pressure and the residue thus obtained was treated with dichloromethane and water. The organic layer was separated, dried over anhydrous sodium sulphate, filtered and concentrated under reduced pressure. The residue thus obtained was purified by column chromatography using 50% ethyl acetate in hexane as eluent to furnish the title compound. Yield: 0.050 g. ¹H NMR (CDCl₃, 400 MHz): δ 7.96 (brs, 1H, -NH & D₂O exchangeable), 7.28-7.24 (m, 2H, Ar-H), 7.15-7.11 (m, 2H, Ar-H), 6.33-6.32 (brs, 1H, -NH & D₂O exchangeable) 5.86-5.85 (d, 1H, J = 4.00 Hz, -CH), 4.75-4.73 (m, 2H, 2x -CH), 4.40-4.39 (m, 1H, -CH), 4.17-4.16 (m, 1H, -CH), 4.00-3.96 (m, 1H, -CH), 3.87-3.84 (m, 1H, -CH), 1.62-1.43 (brm, 10H, 5x -CH₂), 1.36 (s, 3H, -CH₃) and 1.34 (s, 3H, -CH₃).
Mass (m/z, +ve ion mode): 453 [M⁺+1].
Following compounds were prepared analogously,
1,2 -Dioxa spiro[2,5]octane-3-deoxy-3-[(4-trifluoromethylphenyl)-urido]-5,6-O-isopropylidene-α-D-allofuranoside (Compound No. 18);
Mass (m/z, +ve ion mode): 487 [M⁺+1];
1,2-Dioxa spiro[2,5]octane-3-deoxy-3-(phenyl-thiourido)-5,6-O-isopropylidene-α-D-allofuranoside (Compound No. 21);
Mass (m/z, +ve ion mode): 435 [M⁺+1];
1,2-Dioxa spiro[2,5]octane-3-deoxy-3-[(4-cyanophenyl)-thiourido]-5,6-O-isopropylidene-α-D-allofuranoside (Compound No. 22);
Mass (m/z, +ve ion mode): 460 [M⁺+1];
1,2-Dioxa spiro[2,5]octane-3-deoxy-3-[(4-trifluoromethylphenyl)-thiourido]-5,6-O-isopropylidene-α-D-allofuranoside (Compound No. 23);
Mass (m/z, +ve ion mode): 503 [M⁺+1];
1,2-Dioxa spiro[2,5]octane-3-deoxy-3-[(2-trifluoromethylphenyl)-thiourido]-5,6-O-isopropylidene-α-D-allofuranoside (Compound No. 24);
Mass (m/z, +ve ion mode): 503 [M⁺+1].
1,2-Dioxa spiro[2,5]octane-3-deoxy-3-(phenyl-urido)-5,6-O-isopropylidene-α-D-allofuranoside (Compound No. 16);
1,2-Dioxa spiro[2,5]octane-3-deoxy-3-[(4-cyanophenyl)-urido]-5,6-O-isopropylidene-α-D-allofuranoside (Compound No. 17);
1,2-Dioxa spiro[2,5]octane-3-deoxy-3-[(2-trifluoromethylphenyl)-urido]-5,6-O-isopropylidene-α-D-allofuranoside (Compound No. 19).

### Example 13: Synthesis of 1,2-Dioxa spiro[2,5]octane-3-deoxy-3-[(3-chlorobenzyl)-amido]-5,6-O-isopropylidene-α-D-allofuranoside (Compound No. 25)

To a solution of the 1,2-Dioxa spiro[2,5]octane-3-deoxy-3-[(4-fluorophenyl)-thiourido]-5,6-O-isopropylidene-α-D-allofuranoside obtained from step *d* of Example 12 above (0.090 g, 0.301 mmol) in dry dimethylformamide (3 ml) was added *N*-methylmorpholine (0.0913 g, 0.903 mmol) and 3-chlorophenylacetic acid (0.103 g, 0.602 mmol). The mixture was stirred at room temperature for 10 minutes. To the resulting reaction mixture was added 1-hydroxybenzotriazole (0.0813 g, 0.602 mmol) and stirred the mixture at room temperature for 1 hour. To it was added 1-(3-dimethylaminopropyl)-3-ethylcarbodiimide hydrochloride (0.115 g, 0.602 mmol) at 0 °C and stirred at the same temperature for 1 hour and subsequently at room temperature for 12 hours. The reaction mixture was poured into water, extracted with ethyl acetate, washed with water, dried over anhydrous sodium sulphate, filtered and concentrated under reduced pressure. The residue thus obtained was purified by preparative thin layer chromatography using 50 % ethyl acetate in hexane as eluent to furnish the title compound. Yield: 0.065 g.
¹H NMR (CDCl₃ + D₂O exchange, 400 MHz): δ 7.31-7.27 (m, 3H, Ar-H), 7.20-7.17 (m, 1H, Ar-H), 5.85 (brs, 1H, -NH & D₂O exchangeable), 5.83-5.82 (d, 1H, J = 4.00 Hz, -CH), 4.60-4.59 (m, 1H, -CH), 4.20-4.17 (m, 1H, -CH), 4.10-4.06 (m, 1H, -CH), 4.08-4.04 (m, 1H, -CH), 3.92-3.90 (m, 1H, -CH), 3.59 (s, 2H, -CH₂Ar), 1.66-1.61 (m, 6H, 3x-CH₂), 1.55-1.52 (m, 4H, 2x-CH₂),1.39 (s, 3H, -CH₃), and 1.33 (s, 3H, -CH₃).
Mass (m/z, +ve ion mode): 454 [M⁺+1+2] and 452 [M⁺+1].
Following compounds were prepared analogously,
1,2-Dioxaspiro[2,5]octane-3-deoxy-3-[(3-fluorobenzyl)-amido]-5,6-O-isopropylidene-α-D-allofuranoside (Compound No. 26);
Mass (m/z, +ve ion mode): 436 [M⁺+1];
1,2-Dioxaspiro[2,5]octane-3-deoxy-3-[(4-fluorobenzyl)-amido]-5,6-O-isopropylidene-α-D-allofuranoside (Compound No. 27);
Mass (m/z, +ve ion mode): 436 [M⁺+1];
1,2-Dioxa spiro[2,5]octane-3-deoxy-3-[(2-chloro-4-fluorobenzyl)-amido]-5,6-O-isopropylidene-α-D-allofuranoside (Compound No. 28);
Mass (m/z, +ve ion mode): 472 [M⁺+1+2] and 470[M⁺+1];
1,2-Dioxa spiro[2,5]octane-3-deoxy-3-[(2,5-dimethylbenzyl)-amido]-5,6-O-isopropylidene -α-D-allofuranoside (Compound No. 29);
Mass (m/z, +ve ion mode): 446 [M⁺+1];
1,2-Dioxa spiro[2,5]octane-3-deoxy-3-[(3,4,5-trimethoxyobenzyl)-amido]-5,6-O-isopropylidene-α-D-allofuranoside (Compound No. 30);
Mass (m/z, +ve ion mode): 508 [M⁺+1].

### Pharmacological Activity

The compounds of the present invention were tested in the assays described herein. Standard assays were used to evaluate activity of compounds in present invention on inflammatory cells as well as recombinant human 5-lipoxygenase enzyme. Inhibition of 5-lipoxygenase enzyme or attenuation of A23187-induced release of lipid mediator of neutrophil chemo taxis, leukotriene B4 (LTB₄), was used to evaluate inhibitory effect on neutrophils.

### A23187 induced LTB₄ release

Venous blood was collected from healthy human donors using heparin as an anticoagulant. Neutrophils were isolated from freshly drawn blood after dextran sedimentation and ficoll separation (Eur. J. Biochem. 169, 175, 1987). 180 µl of the neutrophil suspension (0.2x10⁶ cells/ml) was taken and added 19 µL of Hank's Buffer salt solution along with 1µL of the test drug (200 times concentrated) in a 24 well plate and incubated at 37°C for 1 hour. Three minutes before the end of test compound incubation, 0.25 mM Ca⁺⁺/Mg⁺⁺were added. Then, 0.3 µg/ml A23187 (Sigma Chem, USA) was added and incubated for further 10 min at 37 °C. The reaction was stopped by adding 80 µL of cold methanol and centrifuged to remove cell debris *(*J. Pharmacol. Exp. Ther. 297:267, 2001). The samples were analysed for LTB₄ release using LTB₄ ELISA kits (Assay Design Inc., USA). The amount of LTB₄ released was quantified and percent inhibition of LTB₄ release was calculated with respect to the difference between the A23187 stimulated and negative control cells, to compute IC₅₀ values. In vitro data obtained on Compounds Nos. 23, 79, 104 and 111 shows IC₅₀ values of between about 9µM and about 700nM, for example, between about 2.2µM and about 700nM, or between about 950nM and about 700nM.

### Assay for 5-Lipoxygenase Activity

In a 96 well UV-plate, 100 µl of phosphate buffer saline (PBS) containing DTT (200 µM), ATP (100 µM) and calcium chloride (100 µM) was added. To each well 0.5 µl of test drug (200 times concentrated) or vehicle was added, followed by 4 µl of recombinant 5-Lox (3 units/µl) and was incubated at 37°C for 5 min. The reaction was initiated by adding 1 µl of 1 mM freshly prepared arachidonic acid and increase in absorbance was monitored at 236 nm for 10 min. (J. Biol. Chem. 261:11512. 1986). A plot of absorbance verses time curve was prepared and area under curve (AUC) was computed for each well. Percent inhibition of AUC for different treatments was calculated with respect to the difference between the arachidonic acid stimulated and negative control values, to compute IC₅₀ values. Data obtained on the disclosed compounds showed IC₅₀ values of between about 10µM and about 300nM, for example, between about 2µM and about 300nM, or between about 1.3µM and about 300nM, or between about 1.0µM and 300nM.

## Claims

1. The compound of Formula I 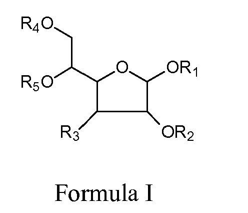 and their pharmaceutically acceptable salts, pharmaceutically acceptable solvates, esters, enantiomers, diastereomers, *N*-oxides, polymorphs, metabolites, wherein
R₁ and R₂ can together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F, Cl, Br, I), aryl, aralkyl, heteroaryl, heterocyclyl, heteroarylalkyl, or heterocyclylalkyl; or R_{L} and Rₘ can together join to form an oxo group].
R₃ can be
A) -(CH₂)ₙG wherein n is an integer from 0-5 and G is selected from
1) NRⱼYRᵤ (wherein Rⱼ is selected from hydrogen, lower (C₁-C₆) alkyl, lower (C₂-C₆) alkenyl, lower (C₂-C₆) alkynyl, lower (C₃-C₆) cycloalkyl, aryl, heteroaryl (with the proviso that the heteroaryl ring is not linked through a heteroatom), aralkyl (C₁-C₄), heteroarylalkyl (C₁-C₄), and heterocyclylalkyl (C₁-C₄),and Y is -C(=O), -C(=S) or SO₂ and Rᵤ is selected from alkyl, alkenyl, alkynyl, cycloalkyl, aryl, aralkyl, heteroaryl, heterocyclyl, heteroarylalkyl, and heterocyclylalkyl; and when n is 0 then Y cannot be -C(=O));
2) -NRⱼC(=T)NRₜRₓ (wherein Rₜ is OH or Rₓ and T is O, S, -N(CN), -N(NO₂), -CH(NO₂), Rⱼ is the same as defined above and Rₓ is selected from hydrogen, alkyl, alkenyl, alkynyl, cycloalkyl, aryl, aralkyl, heteroaryl, heterocyclyl, heteroarylalkyl, heterocyclylalkyl, and -S(O)₂R₇ (wherein R₇ is selected from alkyl, alkenyl, alkynyl, cycloalkyl, aryl, aralkyl, heteroaryl, heterocyclyl, heterocyclylalkyl, heteroarylalkyl, and optionally substituted amino);
R₄ and R₅ can independently be selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier) with the proviso that when R₃ is ORₑ then the acetal must be isopropylidene acetal.

2. A compound selected from the group consisting of:
1,2-O-isopropylidene-3-deoxy-3-[(3-bromobenzyl)-amido]-5,6-dioxa spiro[4,4]nonane-α-D-glucofuranoside (Compound No. 1);
1,2-O-isopropylidene-3-deoxy-3-[(3-chlorobenzyl)-amido]-5,6-dioxa spiro[4,4]nonane-α-D-glucofuranoside (Compound No. 2);
1,2-O-isopropylidene-3-deoxy-3-[(2,4-difluorobenzyl)-amido]-5,6-dioxa spiro[4,4]nonane-α-D-glucofuranoside (Compound No. 3);
1,2-O-isopropylidene-3-deoxy-3-[(2-chloro-4-fluorobenzyl)-amido]-5,6-dioxa spiro[4,4]nonane-α-D-glucofuranoside (Compound No. 4);
1,2-O-isopropylidene-3-deoxy-3-[(3-fluorobenzyl)-amido]-5,6-dioxa spiro[4,4]nonane-α-D-glucofuranoside (Compound No. 5);
1,2-O-isopropylidene-3-deoxy-3-[(3,4,5-trimethoxybenzyl)-amido]-5,6-dioxa spiro[4,4]nonane-α-D-glucofuranoside (Compound No. 6);
1,2-O-isopropylidene-3-deoxy-3-[(2,5-dimethylbenzyl)-amido]-5,6-dioxa spiro[4,4]nonane-α-D-glucofuranoside (Compound No. 7);
1,2-O-isopropylidene-3-deoxy-3-[(3-fluorobenzyl)-amido]-5,6-dioxa spiro[4,4]nonane-α-D-allofuranoside (Compound No. 8);
1,2-O-isopropylidene-3-deoxy-3-[(4-fluorobenzyl)-amido]-5,6-dioxa spiro[4,4]nonane-α-D-allofuranoside (Compound No. 9);
1,2-O-isopropylidene-3-deoxy-3-[(2-chloro-4-fluorobenzyl)-amido]-5,6-dioxa spiro[4,4]nonane-α-D-allofuranoside (Compound No. 10);
1,2-O-isopropylidene-3-deoxy-3-[(3-bromobenzyl)-amido]-5,6-dioxa spiro[4,4]nonane-α-D-allofuranoside (Compound No. 11);
1,2-O-isopropylidene-3-deoxy-3-[(3-chlorobenzyl)-amido]-5,6-dioxa spiro[4,4]nonane-α-D-allofuranoside (Compound No. 12);
1,2-O-isopropylidene-3-deoxy-3-[(2,5-dimethylbenzyl)-amido]-5,6-dioxa spiro[4,4]nonane-α-D-allofuranoside (Compound No. 13);
1,2-O-isopropylidene-3-deoxy-3-[(3,4,5-trimethoxybenzyl)-amido]-5,6-dioxa spiro[4,4]nonane-α-D-allofuranoside (Compound No. 14);
1,2-Dioxa spiro[2,5]octane-3-deoxy-3-[(4-fluorophenyl)-urido]-5,6-O-isopropylidene-α-D-allofuranoside (Compound No. 15);
1,2-Dioxa spiro[2,5]octane-3-deoxy-3-(phenyl-urido)-5,6-O-isopropylidene-α-D-allofuranoside (Compound No. 16);
1,2-Dioxa spiro[2,5]octane-3-deoxy-3-[(4-cyanophenyl)-urido]-5,6-O-isopropylidene-α-D-allofuranoside (Compound No. 17);
1,2 -Dioxa spiro[2,5] octane-3-deoxy-3-[(4-trifluoromethylphenyl)-urido]-5,6-O-isopropylidene-α-D-allofuranoside (Compound No. 18);
1,2-Dioxa spiro[2,5]octane-3-deoxy-3-[(2-trifluoromethylphenyl)-urido]-5,6-O-isopropylidene-α-D-allofuranoside (Compound No. 19);
1,2-Dioxa spiro[2,5]octane-3-deoxy-3-[(4-fluorophenyl)-thiourido]-5,6-O-isopropylidene-α-D-allofuranoside (Compound No. 20);
1,2-Dioxa spiro[2,5]octane-3-deoxy-3-(phenyl-thiourido)-5,6-O-isopropylidene-α-D-allofuranoside (Compound No. 21);
1,2-Dioxa spiro[2,5]octane-3-deoxy-3-[(4-cyanophenyl)-thiourido]-5,6-O-isopropylidene-α-D-allofuranoside (Compound No. 22);
1,2-Dioxa spiro[2,5]octane-3-deoxy-3-[(4-trifluoromethylphenyl)-thiourido]-5,6-O-isopropylidene-α-D-allofuranoside (Compound No. 23);
1,2-Dioxa spiro[2,5]octane-3-deoxy-3-[(2-trifluoromethylphenyl)-thiourido]-5,6-O-isopropylidene-α-D-allofuranoside (Compound No. 24);
1,2-Dioxa spiro[2,5]octane-3-deoxy-3-[(3-chlorobenzyl)-amido]-5,6-O-isopropylidene-α-D-allofuranoside (Compound No. 25);
1,2-Dioxa spiro[2,5]octane-3-deoxy-3-[(3-fluorobenzyl)-amido]-5,6-O-isopropylidene-α-D-allofuranoside (Compound No. 26);
1,2-Dioxa spiro[2,5]octane-3-deoxy-3-[(4-fluorobenzyl)-amido]-5,6-O-isopropylidene-α-D-allofuranoside (Compound No. 27);
1,2-Dioxa spiro[2,5]octane-3-deoxy-3-[(2-chloro-4-fluorobenzyl)-amido]-5,6-O-isopropylidene-α-D-allofuranoside (Compound No. 28);
1,2-Dioxa spiro[2,5]octane-3-deoxy-3-[(2,5-dimethylbenzyl)-amido]-5,6-O-isopropylidene-α-D-allofuranoside (Compound No. 29);
1,2-Dioxa spiro[2,5]octane-3-deoxy-3-[(3,4,5-trimethoxyobenzyl)-amido]-5,6-O-isopropylidene-α-D-allofuranoside (Compound No. 30);
1,2;5,6-Di-O-isopropylidene-3-deoxy-3-(phenyl-propionamido)-α-D-glucofuranoside (Compound No. 31);
1,2;5,6-Di-O-isopropylidene-3-deoxy-3-[(2-methoxyphenyl)-propionamido]-α-D-glucofuranoside (Compound No. 32);
1,2;5,6-Di-O-isopropylidene-3-deoxy-3-[(4-fluorophenyl)-propionamido]-α-D-glucofuranoside (Compound No. 33);
1,2;5,6-Di-O-isopropylidene-3-deoxy-3-(phenyl-propionamido)-α-D-allofuranoside (Compound No. 34);
1,2;5,6-Di-O-isopropylidene-3-deoxy-3-[(2-methoxyphenyl)-propionamido]-α-D-allofuranoside (Compound No. 35);
1,2;5,6-Di-O-isopropylidene-3-deoxy-3-[(4-fluorophenyl)-propionamido]-α-D-allofuranoside (Compound No. 36);
1,2-O-isopropylidene-3-deoxy-3-(phenyl-urido)-α-D-glucofuranoside (Compound No 37);
1,2-O-isopropylidene-3-deoxy-3-[(4-fluorophenyl)-urido]-α-D-glucofuranoside (Compound No. 38);
1,2-O-isopropylidene-3-deoxy-3-[(2-fluorophenyl)-urido]-α-D-glucofuranoside (Compound No. 39);
1,2-O-isopropylidene-3-deoxy-3-[(4-cyanophenyl)-urido]-α-D-glucofuranoside (Compound No. 40);
1,2;5,6-Di-O-isopropylidene-3-deoxy-3-[(2-trifluoromethylphenyl)-urido]-α-D-glucofuranoside (Compound No. 41);
1,2;5,6-Di-O-isopropylidene-3-deoxy-3-[(2-methoxyphenyl)-urido]-α-D-glucofuranoside (Compound No. 42);
1,2;5,6-Di-O-isopropylidene-3-deoxy-3-[(3-fluorophenyl)-thiourido]-α-D-glucofuranoside (Compound No. 43);
1,2;5,6-Di-O-isopropylidene-3-deoxy-3-[(3-trifluoromethylphenyl)-thiourido]-α-D-glucofuranoside (Compound No. 44);
1,2-O-isopropylidene-3-deoxy-3-[(4-cyanophenyl)-thiourido]-α-D-glucofuranoside (Compound No. 45);
1,2-O-isopropylidene-3-deoxy-3-[(3-fluorophenyl)-thiourido]-α-D-glucofuranoside (Compound No. 46);
1,2-O-isopropylidene-3-deoxy-3-[(3-trifluoromethylphenyl)-thiourido]-α-D-glucofuranoside (Compound No. 47);
1,2-O-isopropylidene-3-deoxy-3-[(4-fluorophenyl)-urido]-5,6-dioxa spiro[4,5]decane-α-D-glucofuranoside (Compound No. 48);
1,2-O-isopropylidene-3-deoxy-3-[(2-fluorophenyl)-urido]-5,6-dioxa spiro[4,5]decane-α-D-glucofuranoside (Compound No. 49);
1,2-O-isopropylidene-3-deoxy-3-[(2-trifluoromethylphenyl)-urido]-5,6-dioxa spiro[4,5]decane-α-D-glucofuranoside (Compound No. 50);
1,2-O-isopropylidene-3-deoxy-3-[(4-cyanophenyl)-urido]-5,6-dioxa spiro[4,5]decane-α-D-glucofuranoside (Compound No. 51);
1,2-O-isopropylidene-3-deoxy-3-(octyl-urido)-5,6-dioxa spiro[4,5]decane-α-D-glucofuranoside (Compound No. 52);
1,2-O-isopropylidene-3-deoxy-3-(phenyl-urido)-5,6-dioxa spiro[4,5]decane-α-D-glucofuranoside (Compound No. 53);
1,2-O-isopropylidene-3-deoxy-3-[(2-methoxyphenyl)-urido]-5,6-dioxa spiro[4,5]decane-α-D-glucofuranoside (Compound No. 54);
1,2-O-isopropylidene-3-deoxy-3-[(4-trifluoromethylphenyl)-urido]-5,6-dioxa spiro[4,5]decane-α-D-glucofuranoside (Compound No. 55);
1,2-O-isopropylidene-3-deoxy-3-[(3-methoxyphenyl)-thiourido]-5,6-dioxa spiro[4,5]decane-α-D-glucofuranoside (Compound No. 56);
1,2-O-isopropylidene-3-deoxy-3-[(3-fluorophenyl)-thiourido]-5,6-dioxa spiro[4,5]decane-α-D-glucofuranoside (Compound No. 57);
1,2-O-isopropylidene-3-deoxy-3-[(3-trifluoromethylphenyl)-thiourido]-5,6-dioxa spiro[4,5]decane-α-D-glucofuranoside (Compound No. 58);
1,2-O-isopropylidene-3-deoxy-3-[(3-methoxyphenyl)-urido]-5,6-dioxa spiro[4,5]decane-α-D-glucofuranoside (Compound No. 59);
1,2-O-isopropylidene-3-deoxy-3-[4-fluorophenyl)-urido]-α-D-allofuranoside (Compound No. 60);
1,2-O-isopropylidene-3-deoxy-3-[(2-fluorophenyl)-urido]-α-D-allofuranoside (Compound No. 61);
1,2;5,6-Di-O-isopropylidene-3-deoxy-3-[(4-trifluoromethylphenyl)-urido]-α-D-allofuranoside (Compound No. 62);
1,2;5,6-Di-O-isopropylidene-3-deoxy-3-(phenyl-urido)-α-D-allofuranoside (Compound No. 63);
1,2;5,6-Di-O-isopropylidene-3-deoxy-3-[(2-methoxyphenyl)-urido]-α-D-allofuranoside (Compound No. 64);
1,2;5,6-Di-O-isopropylidene-3-deoxy-3-[4-cyanophenyl)-urido]-α-D-allofuranoside (Compound No. 65);
1,2-O-isopropylidene-3-deoxy-3-[(4-trifluoromethylphenyl)-urido]-α-D-allofuranoside (Compound No. 66);
1,2-O-isopropylidene-3-deoxy-3-(phenyl-urido)-α-D-allofuranoside (Compound No. 67);
1,2-O-isopropylidene-3-deoxy-3-[(2-methoxyphenyl)-urido]-α-D-allofuranoside (Compound No. 68);
1,2-O-isopropylidene-3-deoxy-3-[(4-cyanophenyl)-urido]-α-D-allofuranoside (Compound No. 69);
1,2-O-isopropylidene-3-deoxy-3-[(2-trifluoromethylphenyl)-urido]-α-D-allofuranoside (Compound No. 70);
1,2-O-isopropylidene-3-deoxy-3-[(4-fluorophenyl)-thiourido]-α-D-allofuranoside (Compound No. 71);
1,2-O-isopropylidene-3-deoxy-3-[(2-fluorophenyl)-thiourido]-α-D-allofuranoside (Compound No. 72);
1,2-O-isopropylidene-3-deoxy-3-[(2-trifluoromethylphenyl)-thiourido]-α-D-allofuranoside (Compound No. 73);
1,2-O-isopropylidene-3-deoxy-3-[(2-methoxyphenyl)-thiourido]-α-D-allofuranoside (Compound No. 74);
1,2-O-isopropylidene-3-deoxy-3-(phenyl-thiourido)-α-D-allofuranoside (Compound No. 75);
1,2-O-isopropylidene-3-deoxy-3-[(4-trifluoromethylphenyl)-thiourido]-α-D-allofuranoside (Compound No. 76);
1,2-O-isopropylidene-3-deoxy-3-[(4-cyanophenyl)-thiourido]-α-D-allofuranoside (Compound No. 77);
1,2-O-isopropylidene-3-deoxy-3-[(4-fluorophenyl)-thiourido]-5,6-dioxa spiro[4,5]decane-α-D-allofuranoside (Compound No. 78);
1,2-O-isopropylidene-3-deoxy-3-[(2-fluorophenyl)-thiourido]-5,6-dioxa spiro[4,5]decane-α-D-allofuranoside (Compound No. 79);
1,2-O-isopropylidene-3-deoxy-3-[(2-trifluoromethylphenyl)-thiourido]-5,6-dioxa spiro[4,5]decane-α-D-allofuranoside (Compound No. 80);
1,2-O-isopropylidene-3-deoxy-3-(phenyl-thiourido)-5,6-dioxa spiro[4,5]decane-α-D-allofuranoside (Compound No. 81);
1,2-O-isopropylidene-3-deoxy-3-[(4-trifluoromethylphenyl)-thiourido]-5,6-dioxa spiro[4,5]decane-α-D-allofuranoside (Compound No. 82);
1,2-O-isopropylidene-3-deoxy-3-[(4-cyanophenyl)-thiourido]-5,6-dioxa spiro[4,5]decane-α-D-allofuranoside (Compound No. 83);
1,2-O-isopropylidene-3-deoxy-3-[(2-methoxyphenyl)-urido]-5,6-dioxa spiro[4,5]decane-α-D-allofuranoside (Compound No. 84);
1,2;5,6-Di-O-isopropylidene-3-deoxy-3-[(3-fluorophenyl)-urido]-α-D-allofuranoside (Compound No. 85);
1,2-O-isopropylidene-3-deoxy-3-[(3-fluorophenyl)-urido]-α-D-allofuranoside (Compound No. 86);
1,2-O-isopropylidene-3-deoxy-3-[(3-cyanophenyl)-urido]-α-D-allofuranoside (Compound No. 87);
1,2;5,6-Di-O-isopropylidene-3-deoxy-3-[(3-fluorophenyl)-thiourido]-α-D-allofuranoside (Compound No. 88);
1,2;5,6-Di-O-isopropylidene-3-deoxy-3-[3-trifluoromethylphenyl)-thiourido]-α-D-allofuranoside (Compound No. 89);
1,2-O-isopropylidene-3-deoxy-3-[(4-trifluoromethylphenyl)-urido]-5,6-dioxa spiro[4,5]decane-α-D-allofuranoside (Compound No. 90);
1,2-O-isopropylidene-3-deoxy-3-[(2-trifluoromethylphenyl)-urido]-5,6-dioxa spiro[4,5]decane-α-D-allofuranoside (Compound No. 91);
1,2-O-isopropylidene-3-deoxy-3-[(4-cyanophenyl)-urido]-5,6-dioxa spiro[4,5]decane-α-D-allofuranoside (Compound No. 92);
1,2-O-isopropylidene-3-deoxy-3-[(3-fluorophenyl)-thiourido]-α-D-allofuranoside (Compound No. 93);
1,2-O-isopropylidene-3-deoxy-3-[(3-trifluoromethylphenyl)-thiourido]-α-D-allofuranoside (Compound No. 94);
1,2-O-isopropylidene-3-deoxy-3-[(3-methoxyphenyl)-thiourido]-α-D-allofuranoside (Compound No. 95);
1,2-O-isopropylidene-3-deoxy-3-[(4-fluorophenyl)-urido]-5,6-dioxa spiro[4,5]decane-α-D-allofuranoside (Compound No. 96);
1,2-O-isopropylidene-3-deoxy-3-[(2-fluorophenyl)-urido]-5,6-dioxa spiro[4,5]decane-α-D-allofuranoside (Compound No. 97);
1,2-O-isopropylidene-3-deoxy-3-[(3-fluorophenyl)-urido]-5,6-dioxa spiro[4,5]decane-α-D-allofuranoside (Compound No. 98);
1,2-O-isopropylidene-3-deoxy-3-[(3-cyanophenyl)-urido]-5,6-dioxa spiro[4,5]decane-α-D-allofuranoside (Compound No. 99);
1,2-O-isopropylidene-3-deoxy-3-(phenyl-urido)-5,6-dioxa spiro[4,5]decane-α-D-allofuranoside (Compound No. 100);
1,2;5,6-Di-O-isopropylidene-3-deoxy-3-[(3-methoxyphenyl)-urido]-α-D-allofuranoside (Compound No. 101);
1,2-O-isopropylidene-3-deoxy-3-[(3-methoxyphenyl)-urido]-α-D-allofuranoside (Compound No. 102);
1,2;5,6-Di-O-isopropylidene-3-deoxy-3-[(2-phenyl) propionamido]-α-D-glucofuranoside (Compound No. 103);
1,2;5,6-Di-O-isopropylidene-3-deoxy-3-[3-(1,3-benzodioxol-5-yl) propionamido]-α-D-glucofuranoside (Compound No. 104);
1,2;5,6-Di-O-isopropylidene-3-deoxy-3-[(2-phenyl)-propionamido]-α-D-allofuranoside (Compound No. 105);
1,2;5,6-Di-O-isopropylidene-3-deoxy-3-[3-(1,3-benzodioxol-5-yl)-propionamido]-α-D-allofuranoside (Compound No. 106);
1,2;5,6-Di-O-isopropylidene-3-deoxy-3-(phenyl-urido)-α-D-glucofuranoside (Compound No. 107);
1,2;5,6-Di-O-isopropylidene-3-deoxy-3-[(3-bromobenzyl)-amido]-α-D-glucofuranoside (Compound No. 108);
1,2-O-isopropylidene-3-deoxy-3-[(3-bromophenyl)-amido]-α-D-glucofuranoside (Compound No. 109);
1,2;5,6-Di-O-isopropylidene-3-deoxy-3-[(4-fluorophenyl)-amido]-α-D-allofuranoside (Compound No. 110);
1,2;5,6-Di-O-isopropylidene-3-deoxy-3-[(3-fluorobenzyl)-urido]-α-D-allofuranoside (Compound No. 111);
1,2-O-isopropylidene-3-deoxy-3-[(3-fluorobenzyl)-amido]-α-D-allofuranoside (Compound No. 112);
1,2;5,6-Di-O-isopropylidene-3-deoxy-3-[3-(4-cyanophenyl)-4-methyl-2-thioxo-2,3-dihydro-1*H*-imidazol-1-yl]-α-D-allofuranoside (Compound No. 113);
1,2;5,6-Di-O-isopropylidene-3-deoxy-3-[3-(2-fluorophenyl)-4-methyl-2-thioxo-2,3-dihydro-1*H*-imidazol-1-yl]-α-D-allofuranoside (Compound No. 114);
1,2;5,6-Di-O-isopropylidene-3-deoxy-3-[3-(2-methoxyphenyl)-4-methyl-2-thioxo-2,3-dihydro-1*H*-imidazol-1-yl]-α-D-allofuranoside (Compound No. 115);
1,2;5,6-Di-O-isopropylidene-3-deoxy-3-[3-(2,6-dimethylphenyl)-4-methyl-2-thioxo-2,3-dihydro-1*H*-imidazol-1-yl]-α-D-allofuranoside (Compound No. 116);
1,2;5,6-Di-O-isopropylidene-3-deoxy-3-[3-(3-methoxyphenyl)-4-methyl-2-thioxo-2,3-dihydro-1*H*-imidazol-1-yl]-α-D-allofuranoside (Compound No. 117);
1,2;5,6-Di-O-isopropylidene-3-deoxy-3-(3-phenyl-4-methyl-2-thioxo-2,3-dihydro-1*H-*imidazol-1-yl]-α-D-allofuranoside (Compound No. 118);
1,2;5,6-Di-O-isopropylidene-3-deoxy-3-[3-(2-trifluoromethylphenyl)-4-methyl-2-thioxo-2,3-dihydro-1*H*-imidazol-1-yl]-α-D-allofuranoside (Compound No. 119);
1,2;5,6-Di-O-isopropylidene-3-deoxy-3-[3-(3-cyanophenyl)-4-methyl-2-thioxo-2,3-dihydro-1*H*-imidazol-1-yl]-α-D-glucofuranoside (Compound No. 120);
1,2;5,6-Di-O-isopropylidene-3-deoxy-3-[3-(2-nitrophenyl)-4-methyl-2-thioxo-2,3-dihydro-1*H*-imidazol-1-yl]-α-D-glucofuranoside (Compound No. 121);
1,2;5,6-Di-O-isopropylidene-3-deoxy-3-[3-(4-fluorophenyl)-4-methyl-2-thioxo-2,3-dihydro-1*H*-imidazol-1-yl]-α-D-glucofuranoside (Compound No. 122);
1,2;5,6-Di-O-isopropylidene-3-deoxy-3-[3-(2,6-dimethylphenyl)-4-methyl-2-thioxo-2,3-dihydro-1*H*-imidazol-1-yl]-α-D-glucofuranoside (Compound No. 123);
1,2;5,6-Di-O-isopropylidene-3-deoxy-3-(3-phenyl-4-methyl-2-thioxo-2,3-dihydro-1*H-*imidazol-1-yl]-α-D-glucofuranoside (Compound No. 124);
1,2;5,6-Di-O-isopropylidene-3-deoxy-3-[3-(2-methoxyphenyl)-4-methyl-2-thioxo-2,3-dihydro-1*H*-imidazol-1-yl]-α-D-glucofuranoside (Compound No. 125);
1,2;5,6-Di-O-isopropylidene-3-deoxy-3-(3-phenyl-2-thioxo-2,3-dihydro-1*H*-imidazo1-1-yl]-α-D-glucofuranoside (Compound No. 126);
1,2;5,6-Di-O-isopropylidene-3-deoxy-3-[(4-fluorobenzyl)-thioamido]-α-D-allofuranoside (Compound No. 127);
1,2;5,6-Di-O-isopropylidene-3-deoxy-3-[(2-chloro-4-fluorobenzyl)-thioamido]-α-D-allofuranoside (Compound No. 128);
1,2;5,6-Di-O-isopropylidene-3-deoxy-3-[(2-methoxybenzyl)-thioamido]-α-D-allofuranoside (Compound No. 129);
1,2;5,6-Di-O-isopropylidene-3-deoxy-3-[(3-cyanophenyl)-thiourido]-α-D-glucofuranoside (Compound No. 130);
1,2;5,6-Di-O-isopropylidene-3-deoxy-3-[(2-nitrophenyl)-thiourido]-α-D-glucofuranoside (Compound No. 131);
1,2;5,6-Di-O-isopropylidene-3-deoxy-3-[(2,6-dimethylphenyl)-thiourido]-α-D-glucofuranoside (Compound No. 132);
1,2;5,6-Di-O-isopropylidene-3-deoxy-3-[(2-methoxyphenyl)-thiourido]-α-D-glucofuranoside (Compound No. 133); and
1,2;5,6-Di-O-isopropylidene-3-deoxy-3-[3-(4-fluorohenyl)-4-methyl-2-thioxo-2,3-dihydro-1*H*-imidazol-1-yl]-α-D-allofuranoside (Compound No. 134).

3. A method of making compounds of Formula X, VIIb and VIIc and their pharmaceutically acceptable salts, pharmaceutically acceptable solvates, esters, enantiomers, diastereomers, *N*-oxides, polymorphs or metabolites, wherein the method comprising
a. reacting a compound of Formula II 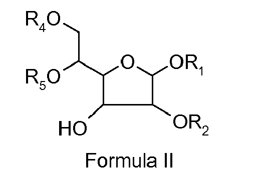 with a compound of Formula IIa
L-hal Formula IIa
to give a compound of Formula III; 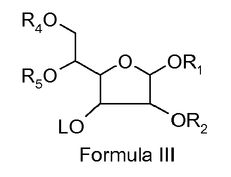
b. reacting a compound of Formula III with sodium azide to form a compound of Formula IV; 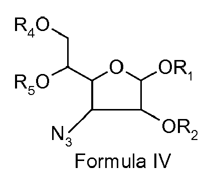
c. reducing a compound of Formula IV to form a compound of Formula V; 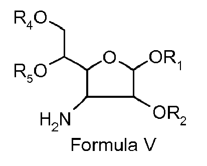
d. reacting a compound of Formula V with a compound of Formula VI
Rᵤ(CH₂)_{f1} COOH Formula VI
or with a compound of Formula VIa
(X=C)=NRᵤ Formula Vla
to furnish a compound of Formula VII; 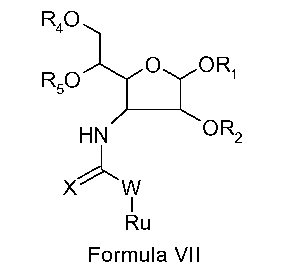
e. hydrolyzing a compound of Formula VII to give a compound of Formula VIII; and 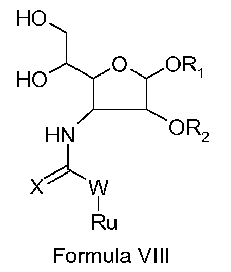
f. reacting a compound of Formula VIII with a compound of Formula IX 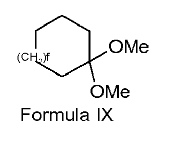 to give a compound of Formula X, 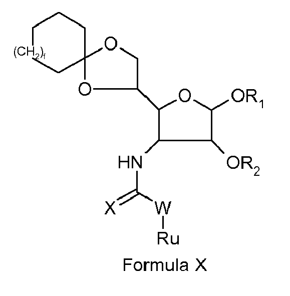 or
reacting a compound of Formula VII (when X = S) 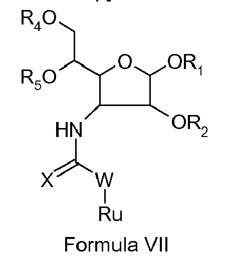 with a compound of Formula VIIa
B₁CH₂COB Formula VIIa
to give a compound of Formula VIIb 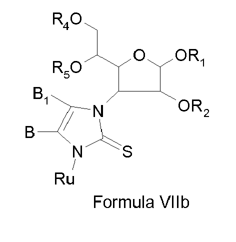 or
thionation of a compound of Formula VII (when X = O) to give a compound of Formula VIIc, 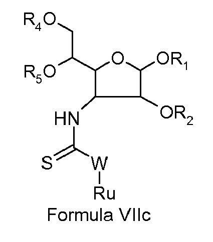 wherein
Rᵤ is selected from alkyl, alkenyl, alkynyl, cycloalkyl, aryl, aralkyl, heteroaryl, heterocyclyl, heteroarylalkyl, and heterocyclylalkyl;
f is an integer from 0-2;
f1 is an integer from 1-3;
L is a leaving group such as tosyl, triflyl or mesyl and hal is a halogen (Cl, Br, I);
W is -CH₂ or -NH₂;
X is oxygen or sulphur;
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F, Cl, Br, I), aryl, aralkyl, heteroaryl, heterocyclyl, heteroarylalkyl, or heterocyclylalkyl; or R_{L} and Rₘ can together join to form an oxo group];
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier); and
B and B₁ are independently selected from the group consisting of alkyl, alkenyl, alkynyl, aryl, heteroaryl, heterocyclyl, cycloalkyl, heterocyclylalkyl or heteroarylalkyl.

4. A method of making a compound of Formula X, VIIb and VIIc and their pharmaceutically acceptable salts, pharmaceutically acceptable solvates, esters, enantiomers, diastereomers, *N*-oxides, polymorphs or metabolites,
wherein the method comprises:
a. oxidizing a compound of Formula II 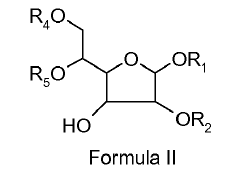 to form a compound of Formula XI; 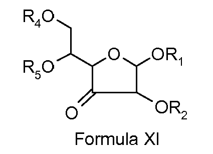
b. reacting a compound of Formula XI with hydroxylamine hydrochloride to form a compound of Formula XII; 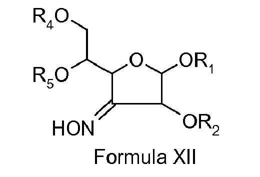
c. reducing a compound of Formula XII to form a compound of Formula V; 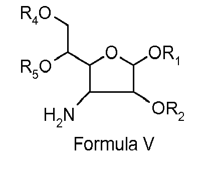
d. reacting a compound of Formula V with a compound of Formula VI
Rᵤ(CH₂)f1 COOH Formula VI
or with a compound of Formula VIa
(X=C)=NRᵤ Formula VIa
to furnish a compound of Formula VII; 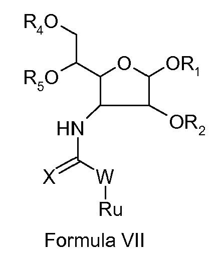
e. hydrolyzing a compound of Formula VII to give a compound of Formula VIII; 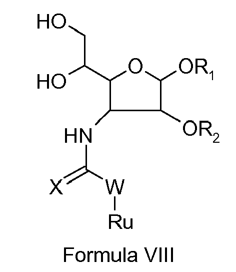
f. reacting a compound of Formula VIII with a compound of Formula IX 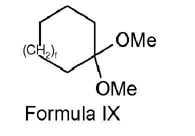 to give a compound of Formula X, 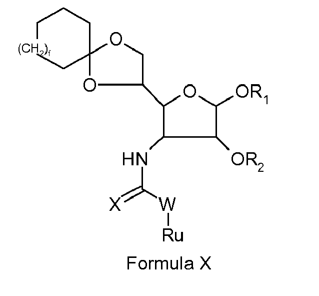 or
reacting a compound of Formula VII (when X = S) 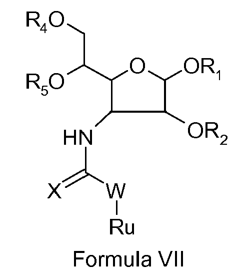 with a compound of Formula VIIa
B₁CH₂COB Formula VIIa
to give a compound of Formula VIIb, 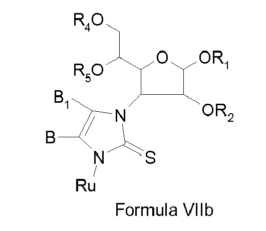 or
thionation of compound of Formula VII (when X = 0) to give a compound of Formula VIIc, 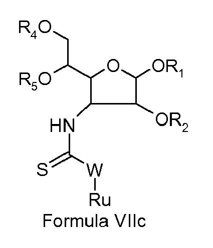 wherein
Rᵤ is selected from alkyl, alkenyl, alkynyl, cycloalkyl, aryl, aralkyl, heteroaryl, heterocyclyl, heteroarylalkyl, and heterocyclylalkyl;
f1 is an integer from 1-3;
f is an integer from 0-2;
W is -CH₂ or -NH₂;
X is oxygen or sulphur;
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F, Cl, Br, I), aryl, aralkyl, heteroaryl, heterocyclyl, heteroarylalkyl, or heterocyclylalkyl; or R_{L} and Rₘ can together join to form an oxo group];
R₄ and R₅ are independently selected from hydrogen, lower (C₁-C₆) alkyl, lower (C₂-C₆) alkenyl, lower (C₂-C₆) alkynyl, lower (C₃-C₈) cycloalkyl, aryl, acyl, heterocyclyl, heteroaryl, lower (C₁-C₄) heterocyclylalkyl, and lower (C₁-C₄) heteroarylalkyl; or R₄ and R₅ may together form a five-membered acetal wherein the carbon linking the two oxygens is substituted with R_{L} and Rₘ (wherein R_{L} and Rₘ are the same as defined earlier); and
B and B₁ are independently selected from the group consisting of alkyl, alkenyl, alkynyl, aryl, heteroaryl, heterocyclyl, cycloalkyl, heterocyclylalkyl or heteroarylalkyl.

5. A method of making a compound of Formula XVIIIa and XIX and their pharmaceutically acceptable salts, pharmaceutically acceptable solvates, esters, enantiomers, diastereomers, *N*-oxides, polymorphs or metabolites,
wherein the method comprises:
a. cyclizing a compound of Formula XIII 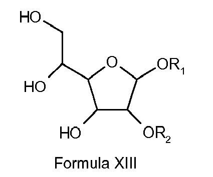 with a compound of Formula XIIIa 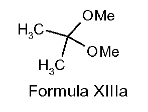 to give a compound of Formula XIV; 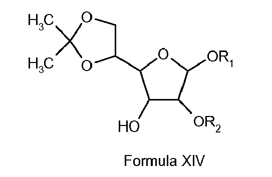
b. oxidizing a compound of Formula XIV to give a compound of Formula XV; 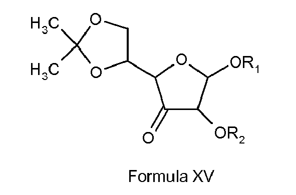
c. reacting a compound of Formula XV with hydroxyl amine hydrochloride to give a compound of Formula XVI; 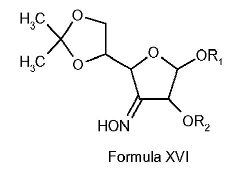
d. reducing a compound of Formula XVI to give a compound of Formula XVII; 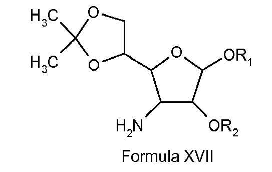
e. reacting a compound of Formula XVII with a compound of Formula VI
Rᵤ(CH₂)_{f1}COOH Formula VI
f. to give a compound of Formula XVIIIa, 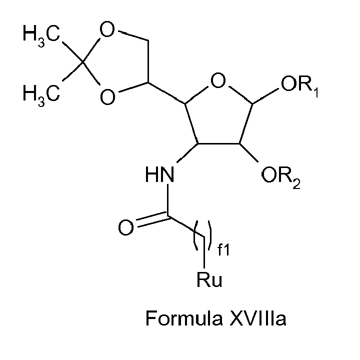 or
g. reacting a compound of Formula XVII with a compound of Formula XVIII
(X=)C=NRₓ Formula XVIII
to give a compound of Formula XIX, 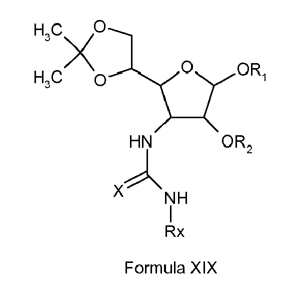 wherein
Rᵤ is selected from alkyl, alkenyl, alkynyl, cycloalkyl, aryl, aralkyl, heteroaryl, heterocyclyl, heteroarylalkyl, and heterocyclylalkyl;
X is oxygen or sulphur;
Rₓ is selected from hydrogen, alkyl, alkenyl, alkynyl, cycloalkyl, aryl, aralkyl, heteroaryl, heterocyclyl, heteroarylalkyl, heterocyclylalkyl, and -S(O)₂R₇;
R₇ is selected from alkyl, alkenyl, alkynyl, cycloalkyl, aryl, aralkyl, heteroaryl, heterocyclyl, heterocyclylalkyl, heteroarylalkyl, and optionally substituted amino;
f1 is an integer from 1-3; and
R₁ and R₂ together form a five-membered acetal, wherein the carbon atom joining the oxygens can be substituted with R_{L} and Rₘ [wherein R_{L} and Rₘ are independently selected from hydrogen, alkyl, alkenyl, alkynyl, cycloalkyl, aryl, or aralkyl; or R_{L} and Rₘ can together join to form a 3-8 membered ring, wherein the ring may optionally contain one or more heteroatoms selected from O, N or S, and the ring may be optionally substituted with one or more of alkyl, alkenyl, alkynyl, amino, substituted amino, cycloalkyl, oxo, hydroxy, carboxy, -COQR₆ (wherein Q is O or NH and R₆ is selected from alkyl, alkenyl, alkynyl, aryl, aralkyl, and cycloalkyl), alkoxy, aryloxy, halogen (F,C1, Br, I), aryl, aralkyl, heteroaryl, heterocyclyl, heteroarylalkyl, or heterocyclylalkyl; or R_{L} and Rₘ can together join to form an oxo group].

6. A pharmaceutical composition comprising a compound of claim 1 and a pharmaceutically acceptable carrier.

7. The use of compounds according to claim 1 for the manufacture of medicament for treating or preventing inflammation, cardiovascular, cancer or autoimmune diseases in mammal.

8. The use of compounds according to claim 1 for the manufacture of medicament for treating or preventing bronchial asthma, chronic obstructive pulmonary disorder, rheumatoid arthritis, type I diabetes, multiple sclerosis, allograft rejection, cancer, inflammatory bowel disease, ulcerative colitis, psoriasis, acne, atherosclerosis, pruritis, allergic rhinitis in mammal.

9. The use of compounds according to claim 1 for the manufacture of medicament for treating or preventing disease or disorder which is mediated through 5-lipoxygenase in mammal.

10. The use of pharmaceutical composition according to claim 6 for the manufacture of medicament for treating or preventing inflammation, cardiovascular, cancer or autoimmune diseases in mammal.

11. The use of pharmaceutical composition according to claim 6 for the manufacture of medicament for treating or preventing bronchial asthma, chronic obstructive pulmonary disorder, rheumatoid arthritis, type I diabetes, multiple sclerosis, allograft rejection, cancer, inflammatory bowel disease, ulcerative colitis, psoriasis, acne, atherosclerosis, pruritis, allergic rhinitis in mammal.

12. The use of pharmaceutical composition according to claim 6 for the manufacture of medicament for treating or preventing disease or disorder which is mediated through 5-lipoxygenase in mammal.
